# EUROPEAN PATENT APPLICATION

(11) **EP 2 444 483 A2**
(43) Date of publication of application: **25.04.2012**
(21) Application number: 11158907.3
(22) Date of filing: 15.05.2006
(51) Int. Cl.: C12N 5/071

(54) **Materials and methods relating to cell based therapies**

(30) Priority: 13.05.2005 GB 0509748
(62) Divisional of application: 06727122.1
(71) Applicant: THE UNIVERSITY COURT OF THE UNIVERSITY OF GLASGOW, Glasgow, G12 8QQ (GB)
(72) Inventor: Shiels, Paul G., Glasgow G31 2ER (GB); Davies, Wayne, Glasgow G12 8QQ (GB)
(74) Representative: Sharples, Andrew John

(57) **Abstract**

The invention provides a novel multipotent cell population of adult origin that can be used to treat ageing and disease, particularly by transplantation to site of cellular damage.

## Description

### The Field of the Invention

The present invention relates to the provision of a cell based therapy for tissue regeneration and the treatment of ageing and disease states associated with cell degeneration or age related tissue changes. This therapy embodies cells of adult origin that can be used in transplantation. The invention further relates to preparations for use in transplantation and tissue regeneration for mitigating cellular and organ damage, and still further, to methods of assessing and the suitability of cell based therapeutic agents for transplantation.

### Background of the Invention

Stem cells are unspecialised cells that have the capacity to proliferate for long periods in culture and which can be induced to become specialized cell types. Stem cells can be isolated primarily from the embryo or adult though these appear to have distinct character and function.

Stem cells have been isolated from the embryos (embryonic/embryo stem cells-ESCs) of numerous mammalian species. Those from the mouse have been subject of intense study for the over twenty years and paved the way for the isolation of human ESCs which have been isolated and worked on since 1998 (1,2).

ESCs are typically derived from the embryonic blastocyst where in vivo they go on to give rise to all subsequent developmental cell types. Adult stem cells (ASCs) on the other hand, are present in numerous tissues where they enable replacement for cells lost through insult and, or, wear and tear.

These cells have the potential to provide cellular based therapies for the treatment of diseases such as Diabetes and Parkinson's disease, where there is cell loss and damage leading to the specific pathology. They also have great potential for screening drugs, toxicological investigations, investigating developmental programming, treating age related tissue loss and degeneration. They also have potential for tissue regeneration following surgical removal, insult or as part of cosmetic surgical procedures.

Presently, there are only limited treatments based on stem cell therapies in clinical usage. One of the complicating factors limiting translation of any such ES cell based therapy or non-haematapoetic ASC therapy, is an inability to definitively control the differentiation of stem cells to generate a single cell type and to control the risk of neoplasia from the use of such a cell source. The pertinence of the latter to ASCs is undetermined. No long term follow-up data is available for any human non-haematapoetic stem cell transplants.

Stem cells have three general properties which distinguishes them from other cells in the body:
(i) They are unspecialised and do not have an apparent specific developmental function.
(ii) They are capable of self-proliferation for long periods in culture, unlike other primary cell types which senesce or undergo Stasis. Stem cells, however, are able to self renew.
(iii)They are able to give rise to specialized cell types with specific functions (e.g.; neurones, beta cells, cardiomyocytes).

Embryonic Stem cells are typically derived from the inner cell mass of the blastocyst of fertilized embryos, a group of approximately 30 cells at one end of the blastocoel. This has led to major moral and ethical debate surrounding the use of such cells derived from human embryos. ESCs are typically isolated through transfer of the inner cell mass to appropriate culture medium in the presence of a murine keratinocyte feeder layer. The resulting culture is subsequently serially passaged for several months to establish a cell line. ESCs so cultured, are typically regarded as a pluripotent cell line after six or more months in culture without differentiation

There is no defined standard test existing to characterise unambiguously cells as ESCs. The typical characterisation relies on a prolonged period of undifferentiated growth in culture and the expression of a marker called Oct 4, in combination with a variety of surface markers (see table 1) which is required for maintenance of self-renewal characteristics. This is correlated with a karyotype analysis to ensure the gross genetic integrity of the cells and a determination of whether the cells can be re-cultured after freeze /thaw cycling.

A further correlation is a determination of pluripotency in vitro and the capacity to form teratomas following injection into an immunosuppressed mouse. Teratomas are benign tumours that routinely contain multiple cell types, thus demonstrating that ESCs are capable of differentiating into multiple cell types (1,2)

Control of undifferentiated ESC growth is well established for those skilled in the art. When, under defined culture conditions, the ESCs are allowed to form embryoid bodies they differentiate spontaneously, to form multiple cell types. This can compromise the production of pure populations of defined cell types for cell base therapies.

Directed differentiation of ESCs, utilising growth in specific culture media, can enrich for particular types of cells. Examples are illustrated in (3,4). Pure cell populations derived from this procedure are potential therapies for Diabetes, Parkinson's disease, spinal cord injury, Duchenne's muscular dystrophy, Heart disease, blindness and many other conditions.

However, the Haematopoetic system has provided a paradigm for ASCs. Based on this, ASCs are thought typically to generate the cell types of the tissue in which they reside, though they have been shown to exhibit plasticity in vitro (5,6) Haematopoetic stem cells (HSCs), for example have been demonstrated to give rise to neurones and cardiomyocytes and demonstrate multi-organ, multi-lineage engraftment (7). This makes ASCs exiting targets for cell based therapies. The term plasticity, as used herein, refers to the ability of a stem cell from one tissue to generate the differentiated cell types of another tissue. This is also referred to as "unorthodox differentiation" (8) or "transdifferentiation" (9,10) in literature in the field.

ASCs are undifferentiated cells found among differentiated cells in an adult tissue or organ, they are unspecialised and can self renew themselves maintaining a capacity to differentiate to yield the major cell types of a tissue or organ. ASCs are considered to maintain and effect tissue repair. The origin of adult stem cells in mature tissues is unknown and the degree of their plasticity remains to be determined. Their use in transplantation is widely known. ASCs from bone marrow have been used in transplants for 30 years. The use of adult non-HSCs, their efficacy, plasticity and safety on long term follow up remains unproven. Reports of non stromal ASCs remains debated in the field, though neural stem cells are now established and accepted as a bone fide ASC type. For a detailed review of the field see (6).

The numbers of ASCs in any tissue appear limited. Characterisation of such cells is still imprecise among those skilled in the art. Typically, this is achieved through testing similar to that used for ESCs, supplemented with other assays. These include labelling the cells in a living tissue with molecular markers and then determining what cell types they generate (30) or taking the cells and ex-vivo labelling them, and following their fate after transplantation into a second animal. Alternatively, the ASCs can be subject to in vitro culture, to determine what differentiated cells types they can give rise to. Clonal isolation and propagation of individual cells followed by transplantation to determination the multi-potential status of the cell type when treating damaged tissue in vivo, is also used (11).

Contemporary hypotheses on ASC differentiation postulate that ASCs may exhibit multipotency and plasticity. Proliferated under the correct growth conditions, they generate the cells and tissues of organs in which they normally occupy.

This can be exemplified by:
(i) Hematopoietic stem cells, which give rise the all the blood cell types
(ii) Bone marrow stromal cells, which give rise to osteocytes, chondrocytes, adipocytes and other kinds of connective tissue cells such as those in tendons.
(iii) Neural stem cells give rise to neurones astrocytes and oligodendrocytes.
(iv) Gut epithelial stem cells which generate absorptive cells, goblet cells, Paneth cells, and enteroendocrine cells in gut crypts.
(v) Skin stem cells which generate keratinocytes, hair follicle and epidermis.

ASCs have also been postulated to transdifferentiate into cells from tissues apart from that in which they reside (10,11). Examples of transdifferentiation include the generation of neural cell types from HSCs, as well as cardiomyocytes and hepatocytes. Cardiomyocytes have also been derived from bone marrow stromal cells, while neuronal stem cells have been used to generate blood and skeletal muscle cells. The mechanism of transdifferentiation and the factors driving it, remain to be determined. This is only one of several key questions still unanswered in the field. It is still undetermined if such plasticity is normal, or an artefact of experimental manipulation. Furthermore, it is still a point of debate in the field as to whether one or more such ASC types actually exist in vivo. The number and types of ASC, where they exist, whether they are left over ESCs, how maintain self proliferation in differentiated tissue and how they eventually differentiate, still remains to be addressed.

The proven pluripotent character of ESCs and the ability to grow them in large numbers makes them attractive candidates for a cell based therapy. This is a severe limitation for the use of ASCs in this context, where such cells are considered rare and their growth conditions not sufficiently defined to produce suitable cells in sufficient numbers for potential therapies.

ASCs do have the critical advantage, however, in that they can be derived from 'self', hence any patient would receive their own cells and not be required to suffer the deleterious side-effects of immuno-suppression to prevent rejection, including a significantly enhanced risk of cancer. Limited potency/plasticity is also considered to be an enhanced safety factor, in that aberrant cell differentiation would be limited and the risk of neoplasia reduced.

ASC based therapies have been proposed to treat conditions such as type 1 diabetes, neurodegenerative disease (including Parkinson's disease and Alzheimer's disease), cardio vascular disease, and osteoporosis. However, many obstacles remain. These include the ability to generate sufficient quantities of cells for transplantation and the ability to differentiate these into the desired cell type(s). These have yet to be shown to integrate and survive in the recipient after transplant. It also has yet to be demonstrated that there long term efficacy following transplant of any cell based therapy and no safety concerns. Furthermore, issues pertaining to graft rejection and cellular damage (pre and post transplant) remain unaddressed.

The use of cellular transplants to combat disease offers great promise for the treatment of conditions such as diabetes and Parkinson's disease. Despite this promise, much controversy exists as to where to isolate suitable cells from, what type of cells can actually be used and what these cells will actually do. Issues pertaining to the safety of cellular transplants using pluripotent cells with the potential for carcinogenesis, still remain.

The use of embryonic and fetal stem cells for example, is also dogged by ethical and moral considerations, but has still managed to provide a wealth of information on stem cell characteristics and potential utilization (1,2).

Cells of adult origin provide an alternative approach which escapes the ethical issues and offers the prospect of cells derived from "self" for transplantation.

Such cells have been termed either, progenitor or stem cells (3) and exhibit a number of characteristics defined for ES stem cells. These cells can be self renewed in culture indefinitely without differentiating into specialized cell types. Furthermore, when grown in suitable medium with appropriate growth factors or morphogens and can be used to derive specialized cell types. Adult stem cells have been isolated from most tissues, but the degree of developmental plasticity exhibited by these cells is open to debate (3).

### Summary of the Invention

Insulin in the pancreas is made by insulin secreting beta cells. In vivo, beta cell turnover is thought to take place throughout life, though controversy exists as to the origin of the replacement cells. Melton et al (30) have provided data indicating that beta cells actually undergo self renewal. Observations in animal models and in vitro experimentation indicate that pancreatic ductal cells may still provide a source of cells able that are analogous to beta cells in that they can produce insulin and give rise to islet like clusters (4). There is also evidence that these cells express the neural stem cell marker nestin (4,12)

The inventors have determined that a population of cells from the adult pancreas can function like ASCs. Accordingly, at its most general, the present invention provides materials and methods for treating diseases and conditions of ageing based on a cell based therapy using a novel multipotent adult cell population termed Pathfinder Cells (PCs). For the first time, this provides a cell based therapy for diseases such as Diabetes and neurodegenerative disorders such as Parkinsons.

PCs derived from adult rat pancreas have been deposited in accordance with the Budapest Treaty 1977 at The European Collection of Cell Cultures, Porton Down, Salisbury Wiltshire, UK, SP4 0JG on 12 May 2005 by The University Court of the University of Glasgow under ECACC No. Q6203.

The inventors have also derived an analogous population of cells from the human breast and human kidney. Further, the inventors have demonstrated the presence of a functionally analogous population of cells from the rodent bone marrow.

The inventors have surprisingly determined that insulin producing cells can be derived from the multipotent adult cell population that could be used for transplantation into diabetic patients. The cell population expresses Nestin when analysed by RT-PCR and they are capable of growth in the absence of extracellular matrix, such as Matrigel^{(™)} free culture system, in the presence of serum in the growth medium.

The inventors have further determined that, following transplantation, the PCs are able to induce host cells to regenerate the damaged pancreatic tissue. Thus, it appears that this novel cell population is not only able to mature into insulin producing cells, but is also able to stimulate host cells to regenerate. This finding has considerable importance in the field of cell based therapies.

In a first aspect of the present invention, there is provided an isolated cell population (stem or progenitor cell-like) originating from adult tissue, wherein said cells are Nestin (RAT:NP_037119.1 GI:6981262; HUMAN: NP_006608.1 GI:38176300) positive (e.g. by PCR) (and preferably PDX-1 negative (HUMAN: NP_000200.1 GI:4557673; RAT: NP_074043.3 GI:50838802) and capable of growth in a Matrigel free culture system with the presence of serum in the growth medium.

The percentage of Nestin positive cells in the population may be less than 50% as determined by flow cyometry analysis. Nevertheless, they are distinguishable as Nestin positive cells by e.g. PCR.

The cell population is also CD90 positive (RAT:LOCUS:P01830 GI135832; NP_006279 GI:19923362) e.g. by flow cytometry. The percentage of CD90 positive cells may be less than 50%.

The adult pancreatic tissue is preferably human. However, it may be derived from other mammalian species e.g. rat, mouse, primates, pig etc.

The adult tissue is preferably pancreatic tissue, but may be tissue derived from other organs such as breast, bone marrow, heart, liver or kidney.

Preferably the cell population comprises cells deposited under accession number Q6203 at ECACC on 12 May 2005.

There is also provided a pharmaceutical composition comprising a cell population in accordance with the invention along with a pharmaceutically acceptable carrier.

In a second aspect of the invention, there is provided a method of isolating a multipotent cell population from adult mammalian tissue, said method comprising
obtaining tissue from adult organs;
culturing said tissue; and
isolating emergent cell population monolayer.

Preferably the tissue is pancreatic and the adult organ is the pancreas (e.g. adult pancreatic ducts). However, the inventors have determined an analogous cell population in breast tissue and kidney tissue. Accordingly, the adult organs and derived tissue may be from the breast, liver or kidney.

Preferably the tissue is cultured in CMRL-1066 medium which is commercially available (Sigma - C-0422). However, other media will be known to the person skilled in the art.

The tissue obtained from adult pancreatic ducts may comprise whole ducts, or more preferably, comprises duct tissue which has been minced to aid in the culturing step.

The invention further provides a adult cell population producible by the above method.

In a third aspect of the invention, there is provided a method of obtaining PC extract, said method comprising growing PC cells of the first aspect of the invention in suitable medium and collecting said medium comprising PC extract.

The PC extract preferably comprises secreted cell factors, cell extracts or combinations thereof.

Accordingly, the invention also provides preparations consisting of medium in which a PC population has grown. The medium may be treated to remove active chemicals/compounds of interest. Further, the PC may be treated directly and their extracts used in producing such a preparation.

In accordance with this embodiment, there is also provided a method of using such preparations or medium to treat disease or conditions of ageing associated with cell loss or degeneration and for tissue regeneration associated with disease or cosmetic procedures.

The medium, preparation, derived PC factors or extracts or combinations thereof may be used in methods of treatment (including cosmetic surgery) by administering directly to an individual.

The invention also provides a method of obtaining cell factors, extracts etc. from the growth medium and testing those factors to determine factors capable of modulating cell growth and differentiation.

In a fourth aspect of the invention, there is provided a method of treating a disease state or condition of ageing associated with cell loss or degeneration, said method comprising the steps of administering a cell population (or cell secreted factor, cell extract or combinations thereof) in accordance with the first aspect to a patient having said disease.

The cells etc. are preferably administered intraveneously or they may be transplanted to the disease site.

Preferably, the disease is associated with degeneration of pancreatic cells, neuronal cell, cardiovascular cells (e.g. cardiomyocytes), epithelial cells, liver cells or kidney cells.

The disease state to be treated may include diabetes (type I and II), liver disease, kidney disease, eye disease, Parkinson's disease and cardiovascular disease and age related degenerative conditions of the organs and tissues of the body. This aspect of the invention may also be used as a form of cosmetic surgery, e.g. cell regeneration for tissues and to prevent forms of ageing.

It will be appreciated that a preferred embodiment of the invention is where the donor of the cells and the recipient are the same species, ideally human. However, the inventors have surprisingly determined that the cells work across the species barrier with little or no immunological problems. Accordingly, an embodiment of the present invention includes the use of rat PCs in the treatment of human patients.

In a fifth aspect of the invention, there is provided a method of producing a specified differentiated cell population, said method comprising the steps of
providing a PC population;
optionally, selecting a PC cell subpopulation using one or more markers provided in Table 3 and others exemplified herein; and
culturing said subpopulation of cells in an environment conducive to cell differentiation.

The above method may also include the step of determining suitability for transplantation of given PC cells and derived populations of differentiated and undifferentiated cells for transplantation comprising
detecting the presence in the cell population of one or more of the markers provided in Table 4.

The invention further provides a cell population in accordance with the first aspect of the invention for use in a method of medical treatment including cosmetic surgery.

There is also provided use of a cell population in accordance with the invention in the preparation of a medicament to treat a disease state or condition of ageing associated with cell loss or degeneration, e.g. diabetes and Parkinson's disease.

Aspects and embodiments of the present invention will now be illustrated, by way of example, with reference to the accompanying figures. Further aspects and embodiments will be apparent to those skilled in the art. All documents mentioned in this text are incorporated herein by reference.
Figure 1. Light photomicrograph of PCs grown in CMRL
Figure 2. Marker characterization of PCs
Figure 2.2 RT-PCR characterization of PCs
Figure 3 TRAP assay for PCs
Figure 4
Figure 5.1
Figure 5.2

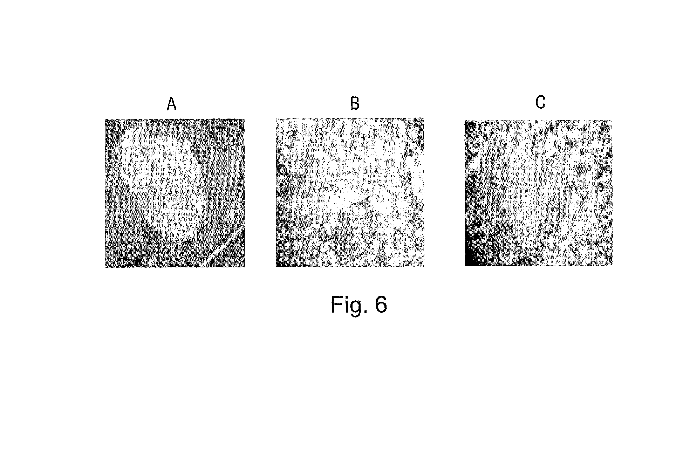

Figure 6. Post mortem analysis of PC treated STZ-diabetic mice

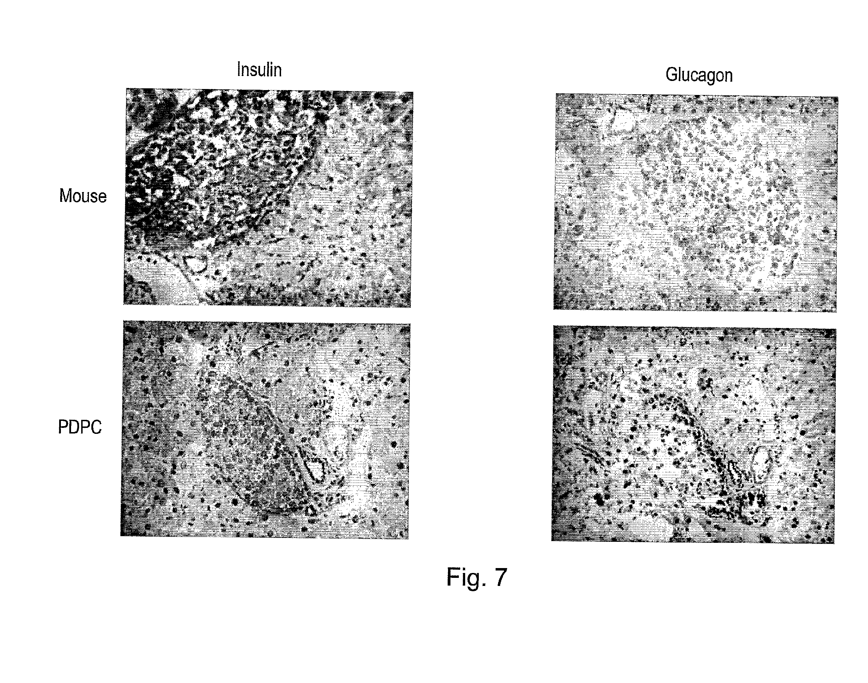

Figure 7. Immunohistochemical staining for insulin and glucagon in PC treated STz-diabetic mice.

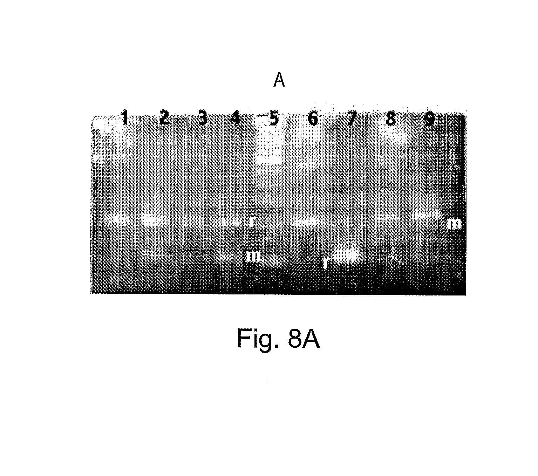

Figure 8A. Hin F1 PCR RFLPs for insulin.

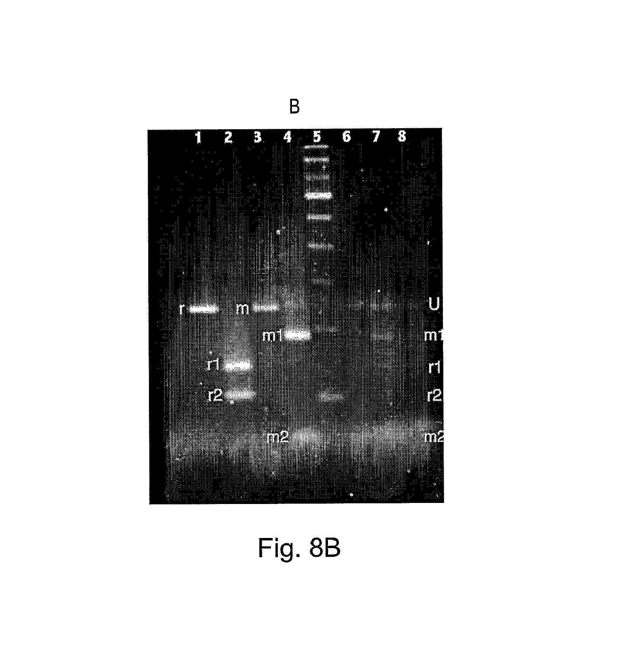

Figure 8b. Hin F1 RFLPs for insulin.

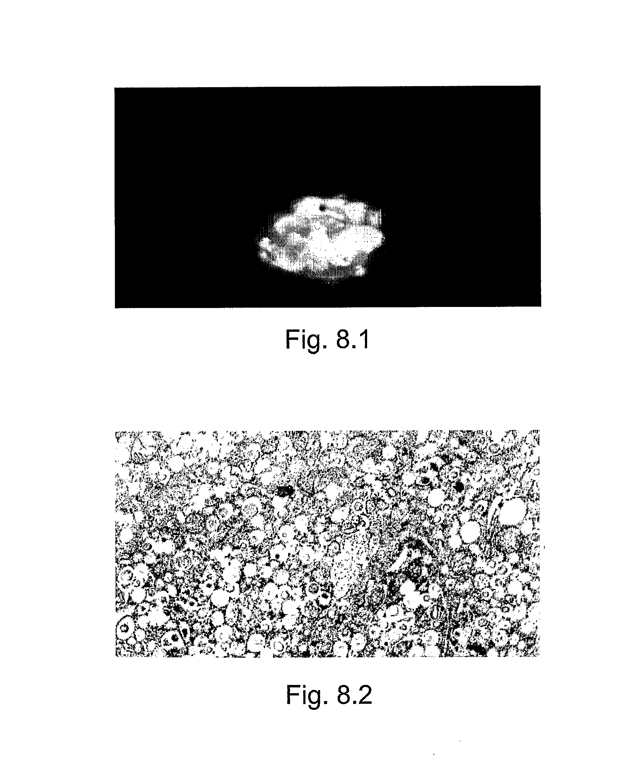

Figure 8.1 Islets derived from PCs

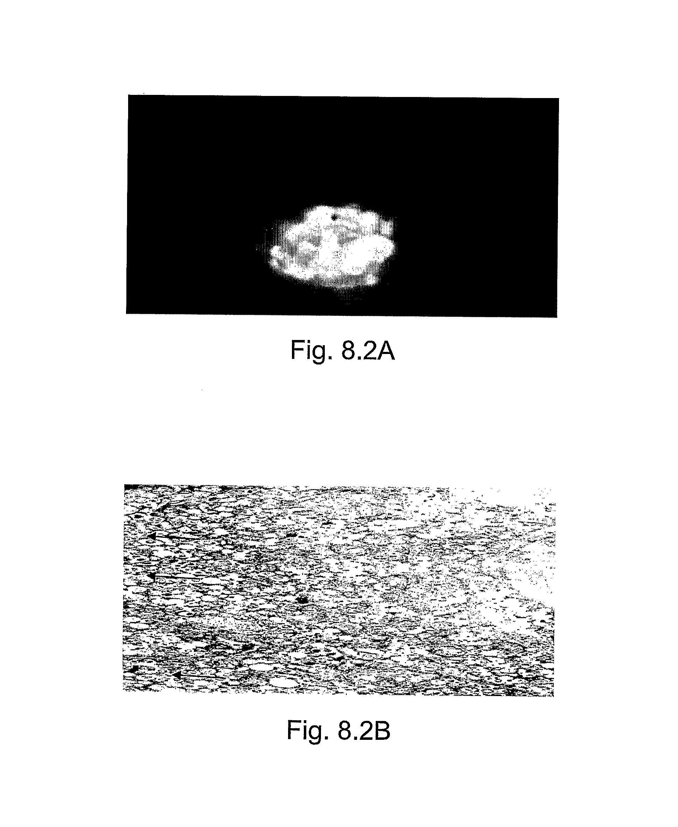

Figure 8.2 Neuronal like cell derived from PC
Figure 8.2A ICH staining of islet like cluster derived from PCs
Figure 8.2B TEM of islet cluster derived from PCs. Examples of dense core vesicles with electron translucent halo are marked with arrows.

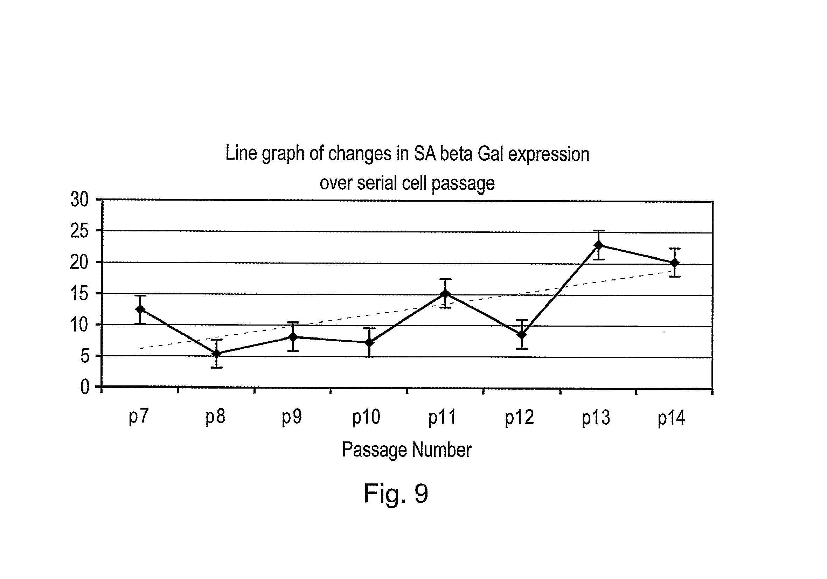

Figure 9. SA β Gal analysis of serially passaged PC cultures.

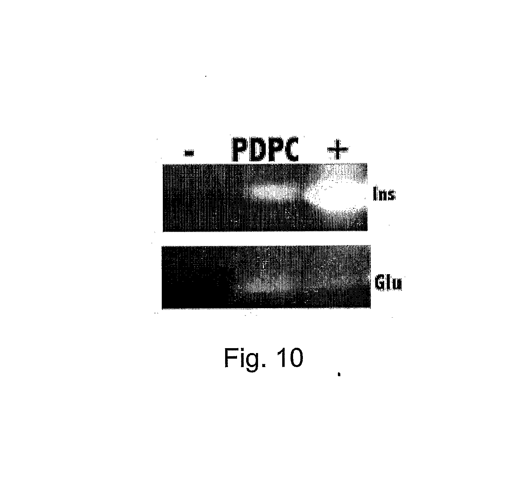

Figure 10. RT PCR analysis of islet markers.

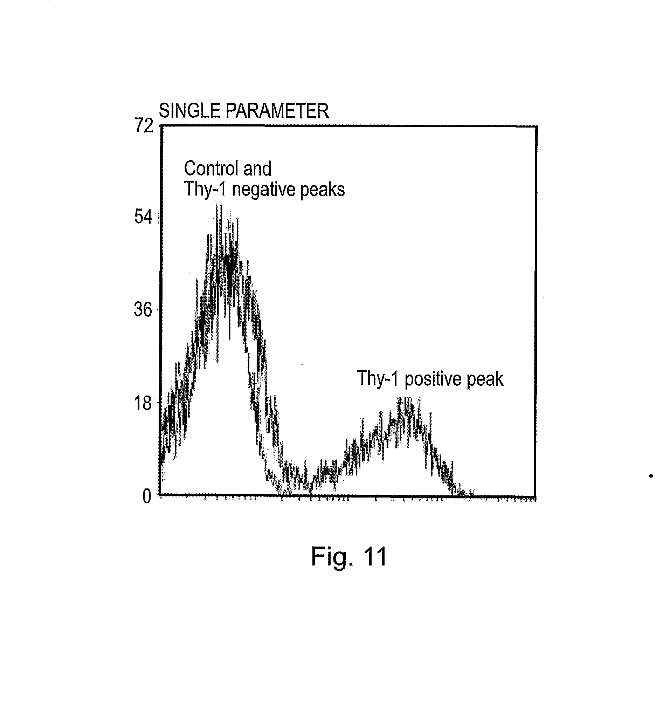

Figure 11. FACs analysis of PCs for Thy-1 expression.

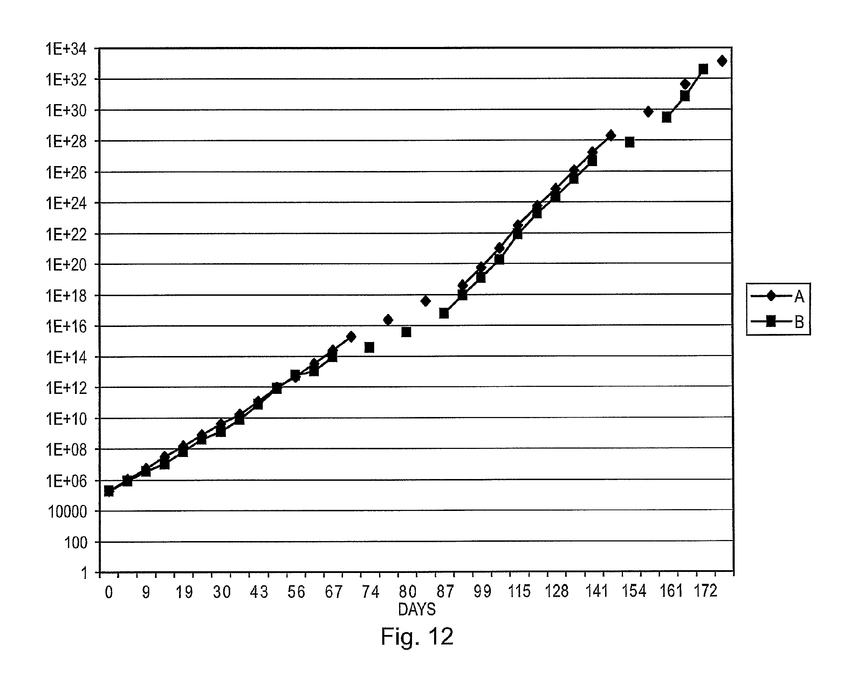

Figure 12. PC growth characteristics in culture.

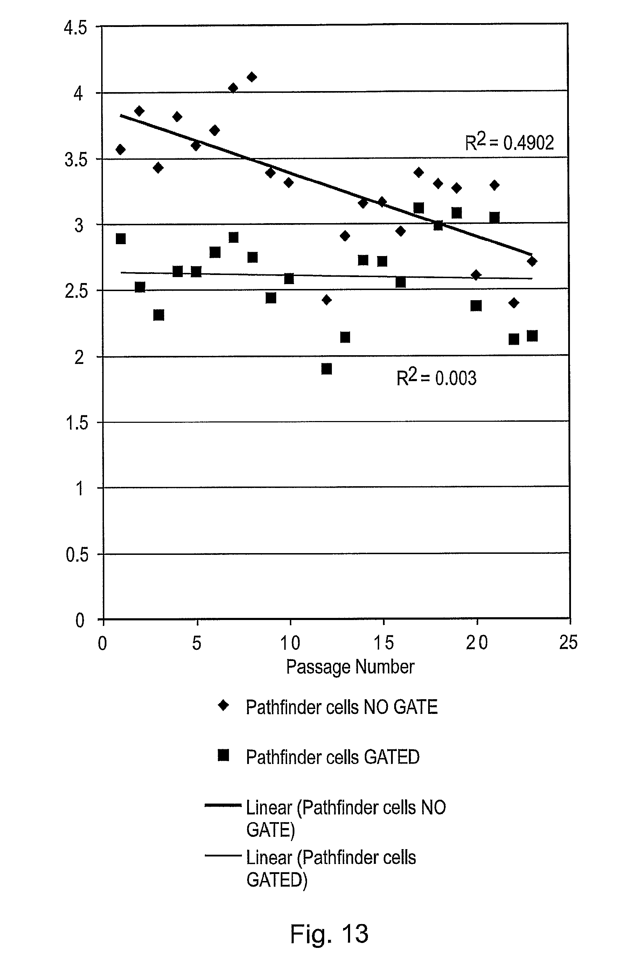

Figure 13. Determination of Lipofuscin content in serially passaged PCs.

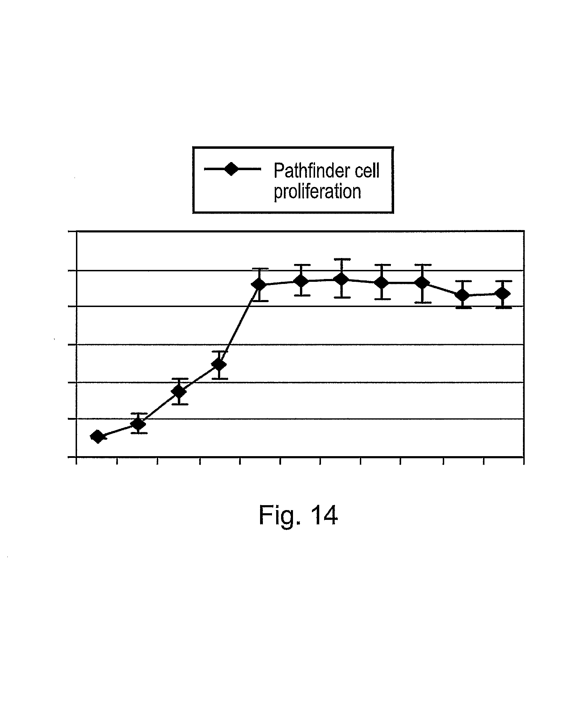

Figure 14. WST analysis of Pathfinder cells with increasing oxidant insult.

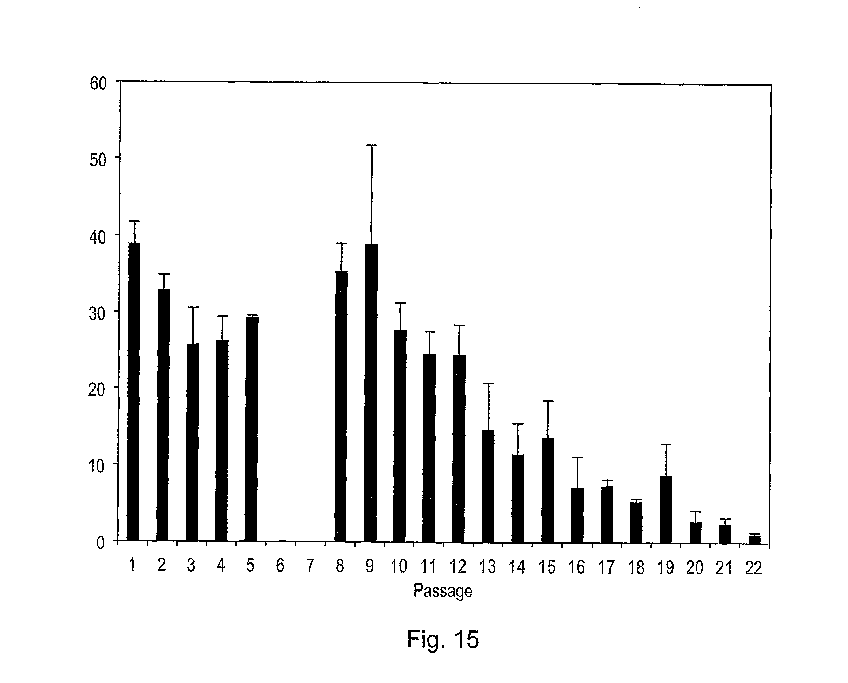

Figure 15. Percentage expression of SA β Gal in PCs with serial passage.

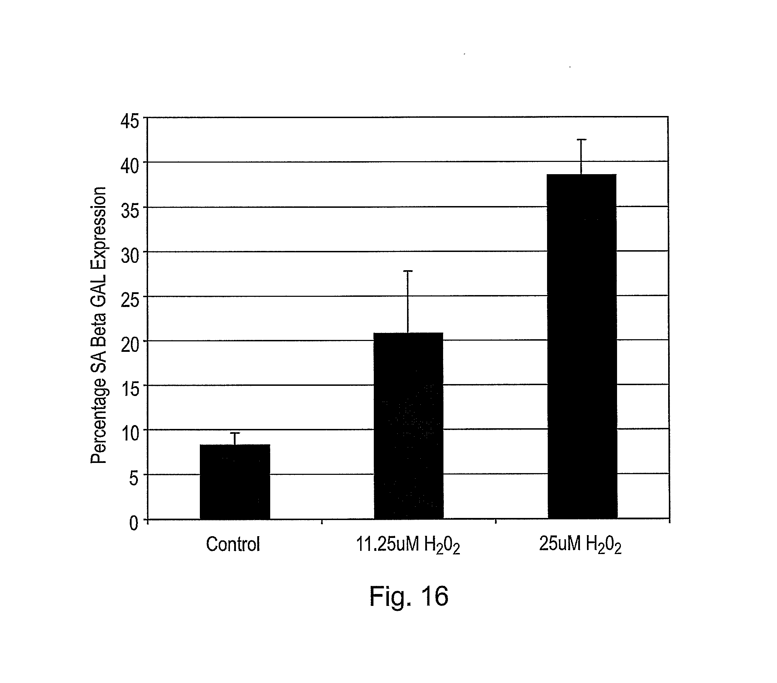

Figure 16. Percentage expression of SA β Gal in PCs after treatment with Hydrogen Peroxide.

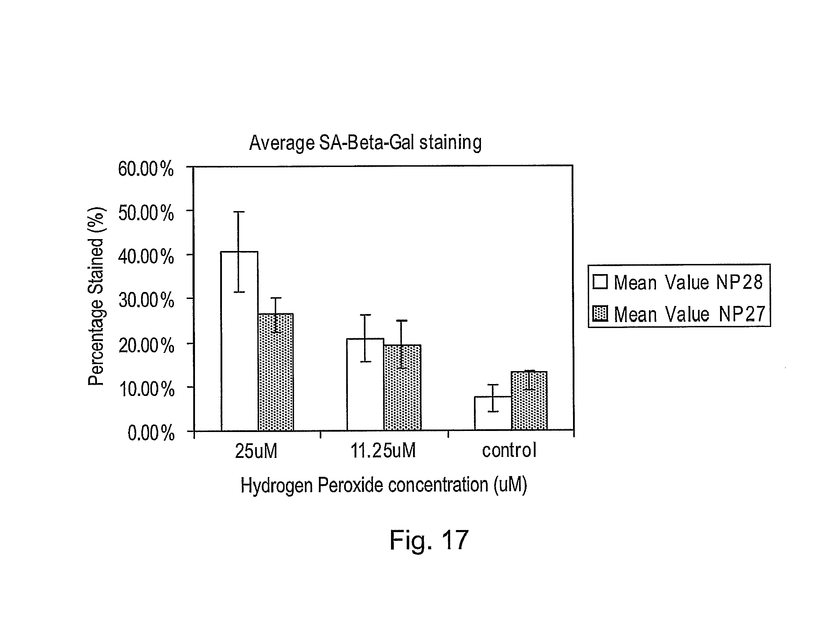

Figure 17. Percentage expression of SA β Gal in PCs over serial passage with acute oxidant challenge.

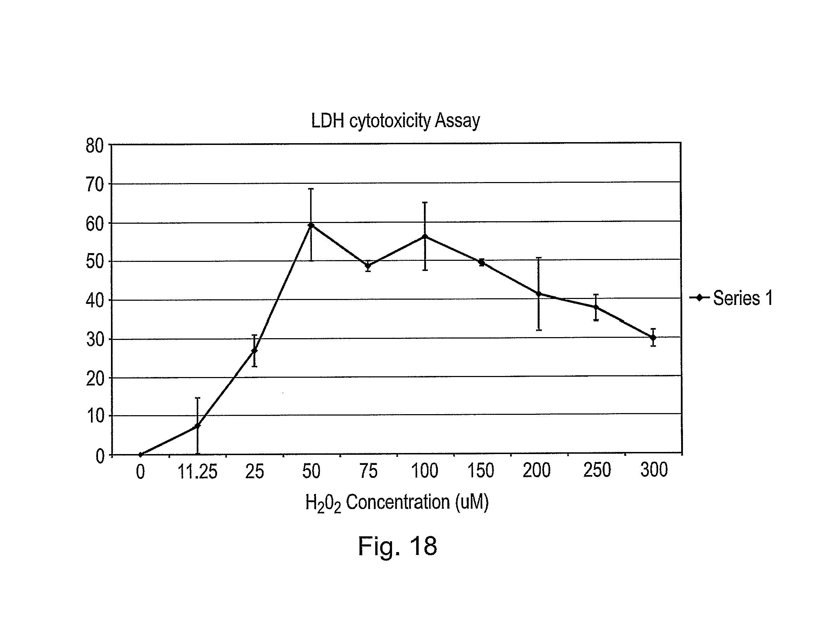

Figure 18. LDH cytotoxity assay for PCs under challenge from H₂O₂.

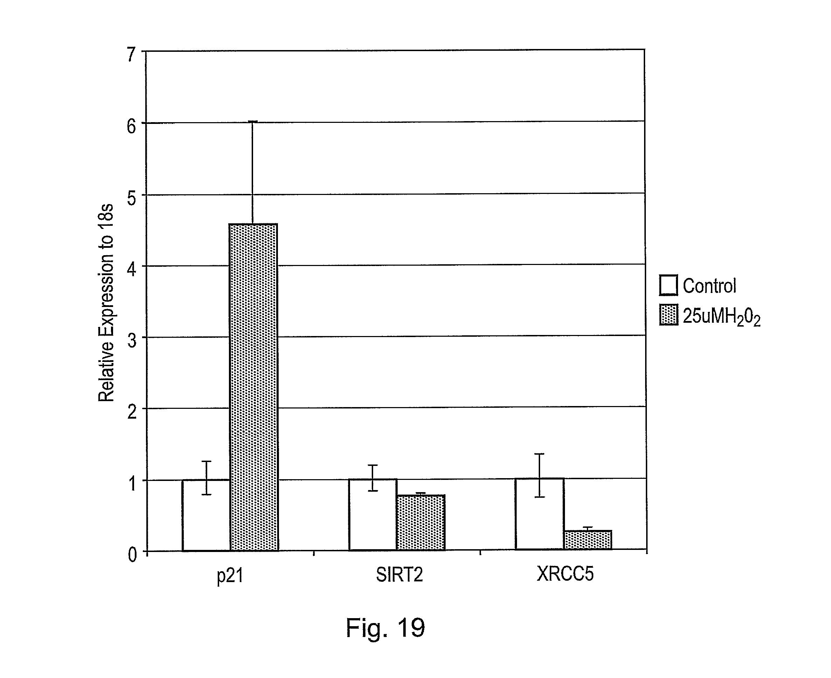

Figure 19. Relative expression of p21, SIRT2 and XRCC5 in PCs after 25µM Hydrogen peroxide treatment.

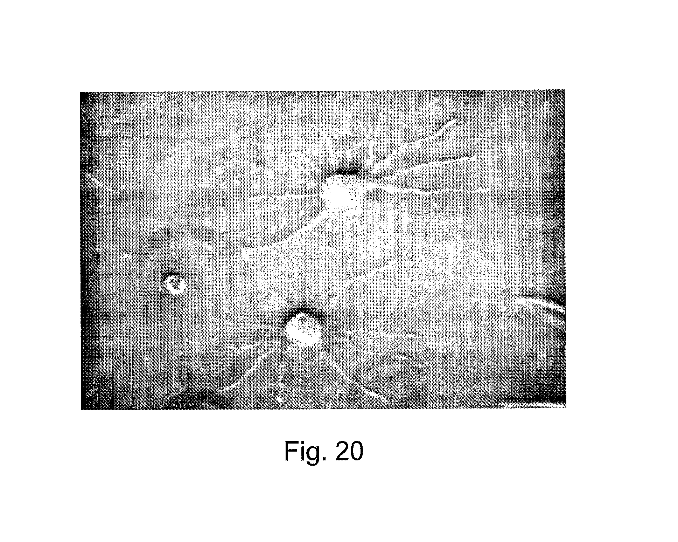

Figure 20. Light micrograph (X100) of differentiated clonal isolate of a PC to generate a neural progenitor cell.

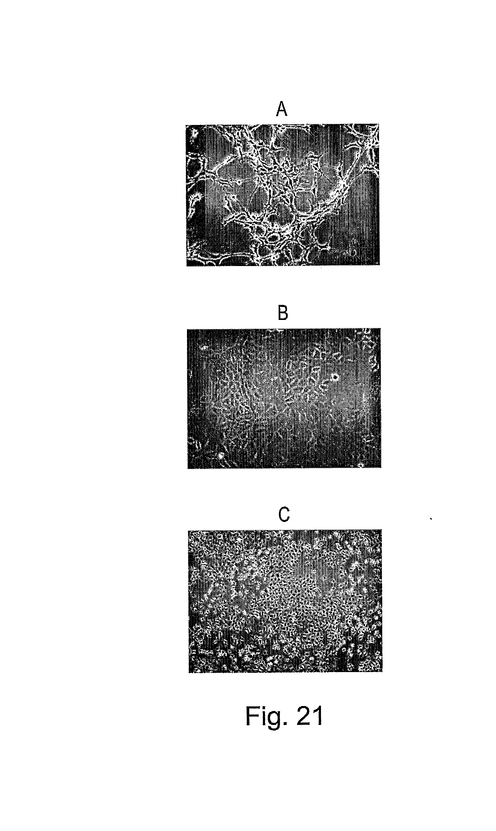

Figure 21. PCs grown in defined hepatocyte differentiation CD90+ (Panel A) and CD90- (Panel B) and undifferentiated cells (Panel C).

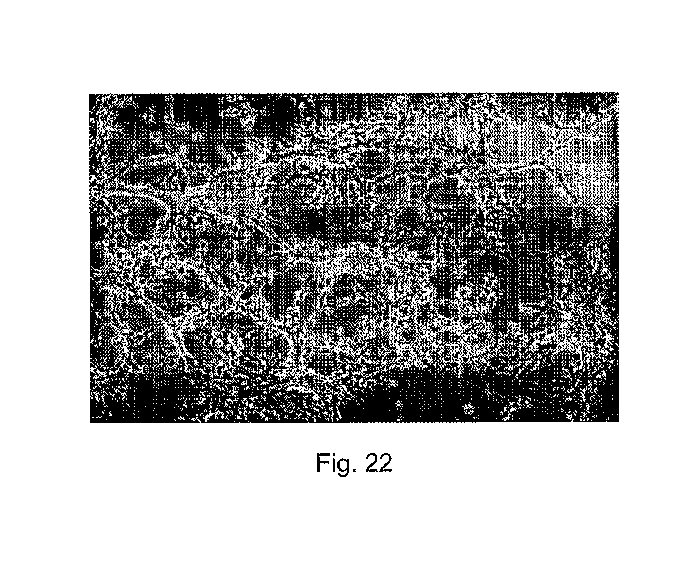

Figure 22. Differentiating CD90 + cells in process of forming islet clusters (indicated by arrows).

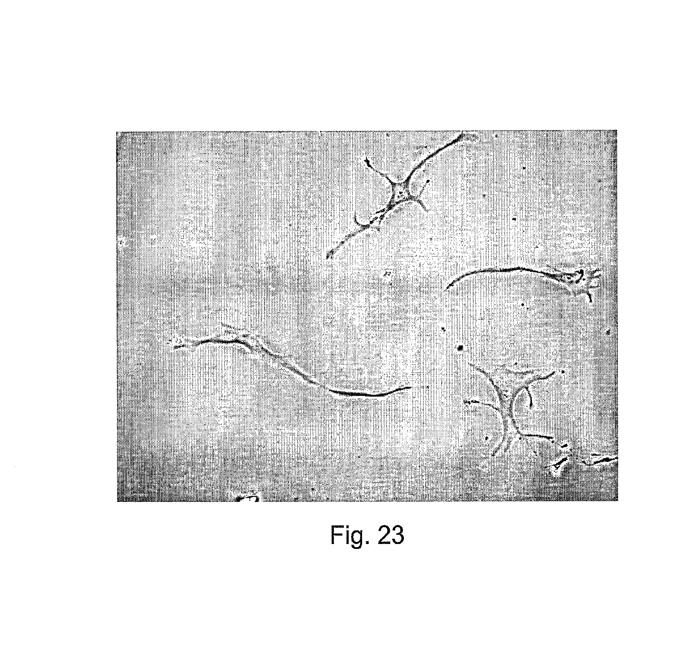

Figure 23. Human breast derived Pathfinder cells (x100).

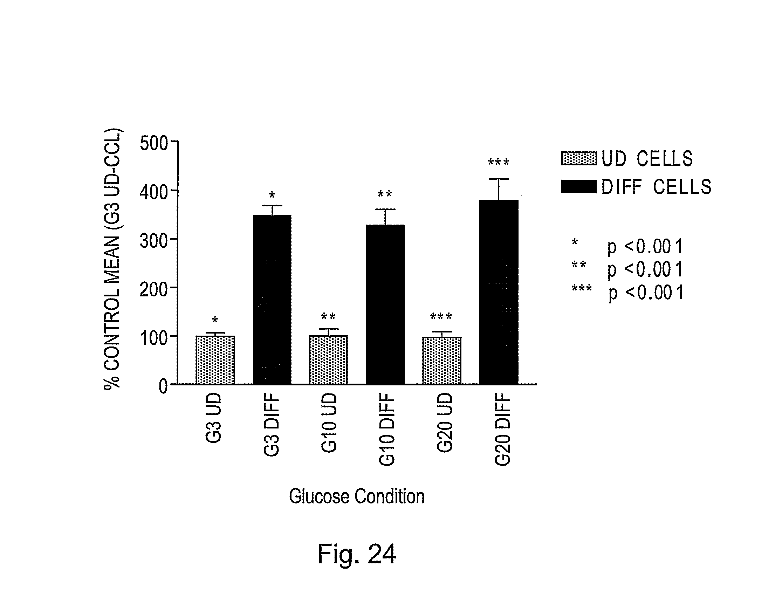

Figure 24. Glucose stimulated insulin secretion (pg) per mg of protein per minute in undifferentiated PCs (UD) and islets derived from PCs (DIFF), stimualted with 3 (G3),10 (G10) and 20 (g20) mM Glucose.

### Example 1

### Mitigation of STZ induced diabetes by adult pancreatic derived pathfinder cells in a rodent xenograft model.

### Introduction

World wide 150 million people suffer from diabetes. Despite insulin therapy late complications such as retinopathy, nephropathy and neuropathy are not uncommon. Transplantation of cadaveric islets offers one means to treat the problem of type I diabetes, but this is compromised through lack of available organs. Stem cell therapies offer a potential means to tackle these problems. It is not yet clear whether this technology will be able to generate fully functional beta cells.

One approach to facilitate the generation of beta cells has involved expansion and differentiation of adult human pancreatic progenitor cells, which are closely related with the beta cell lineage and which would avoid the controversy and technical problems associated with the use of pluripotent stem cells (13). Evidence that stem cells reside in the pancreatic ducts has been provided by rodent models of pancreas regeneration (14,15,16), though the status of stem cells in the adult pancreas is unclear.

In vitro mouse ductal cells have been demonstrated to provide a source of pancreatic progenitors (17,18). Endocrine differentiation has also been reported in cultures enriched for human pancreatic duct cells (19).

The exact nature of any pancreatic progenitor/stem cells is still controversial. In addition to the duct epithelial cell, a further candidate islet progenitor cell has been described, based upon expression of the neural stem cell marker nestin and lack of known islet and duct cell markers (12,20).

Such nestin-positive cells have been reported to differentiate in vitro into pancreatic endocrine, exocrine, and hepatic phenotypes (12). Differentiation of insulin-producing cells from mouse ESCs has also been described as involving a nestin expressing intermediate cell type (21). This is contrary to established descriptive analyses of mouse and human pancreas development, which preclude a role for any nestin expressing cells in islet differentiation (22).

A more traditionally accepted view is that pancreatic endocrine progenitor/stem cells express PDX-1, a known marker for insulin producing cells. (19) Such a cell type has been demonstrated to be amenable to in vitro manipulation and when grown in the presence of fibroblast growth factor-7 (FGF-7) to stimulate ductal cell proliferation. Further growth on Matrigel, supplemented with nicotinamide (NIC) has been reported to initiate and stimulate endocrine differentiation (23-25).

This data has been supported by the identification of a similar human cell population. These cells have been reported to require serum free growth conditions and the use of Matrigel as absolute essentials. They have been induced in vitro to produce islet like structures, which produce insulin in response to glucose stimulation. Critically, no evidence was found for the development of endocrine cells from nestin-positive stem cells (26)

The inventors have isolated multipotent adult stem cells from the pancreas from which to derive to insulin producing cells that could be used for transplantation. Adult cells were sought to evade ethical and moral dilemmas associated with embryonic and fetal cell types. Critically, the inventors have sought to demonstrate that this can be achieved with the use of Nestin positive cells and the presence of growth in a Matrigel free culture system with the presence of serum in the growth medium.

A significant feature of this line of experimentation, is that it was designed to determine the fates of the transplanted cell with respect to any tissue regeneration. Hence, it would be determined if the PCs induced host cells to regenerate the damaged pancreatic tissue, or if they themselves proliferated and regenerated the tissue, or whether it was a combination of both. This is a critical aspect of the current application as it is not considered obvious in the field that host tissue could be regenerated via stimulation of cells from a another species, or indeed that a combination of both species cells could effect tissue repair.

### Materials and Methods

Pancreatic ducts were isolated from 12 month old Albino Swiss rats and minced, prior to seeding in CMRL medium. The Pathfinder cells emerged as a confluent monolayer after approximately 5 weeks in culture. These were then harvested and washed in PBS

### Maintenance of PCs

PCs are maintained in culture in 20mls CMRL 1066 medium (Invitrogen, Paisley, UK.) supplemented with 10% Foetal Bovine Serum (Sigma, Poole, UK), 2mM Glutamax, 1.25ug/ml Amphotericin B, and 100u/ml/100ug/ml Penicillin/Streptomycin, (all Invitrogen, Paisley, UK) in T75 Culture Flasks with 0.2um Filter Caps(Corning, UK) at 37°C in a 5% CO2 atmosphere.

Sub-Confluent cultures are passaged by the total removal of culture medium by pipette and the washing of the adherent cells by the addition of 10mls Calcium and Magnesium-free Hanks Balanced Salt Solution (HBSS), (Cambrex Bio-Science, Wokingham, UK) to the flask for 5 minutes at room temperature. After the removal of the HBSS from the flask by pipette, 2mls of Trypsin-Versene solution (200mg/L Versene, 500mg/L Trypsin) is added to the flask. The flask is periodically examined microscopically until dissociation of the cell monolayer can be confirmed. Cells are then removed by pipette and re-cultured as above at a density of 1/5 → 1/10 as desired by the addition of 20mls of fresh culture medium. The medium should be replaced twice weekly with 20mls of fresh medium. After seceral passages to establish the cell popualtion the PCs are maintained in 20mls CMRL 1066 medium (Invitrogen, Paisley, UK) supplemented with 5% Foetal Bovine Serum (Sigma, Poole,UK), 2 mM Glutamax, 1.25ug/ml Amphotericin B, and 100µ/ml Penicinllin/Streptomycin (all Invitrogen, Paisley) in T75 or T160 flasks with 0.2um filtercaps at 37°C in a 5%CO2 atmosphere.

### Flow Cytometry

Cultured cells are washed and trypsinised as above. They are then centrifuged at 1000 x rpm for 10 minutes and the resulting cell pellet is resuspended in PBS. A Trypan blue (Invitrogen, Paisley, UK) viability count is then undertaken and 100,000 → 1,000,000 cells/ml are then labelled with 100ul of a 1/75 dilution Mouse anti-Rat CD 90 antibody (Serotec, Kidlington, UK)in 0.2% BSA/PBS for 45 minutes at 4°C in the dark. They are then washed and centrifuged three times at 1000 x rpm in 0.2% BSA/PBS before being labelled by the addition of 100ul of a 1/15 dilution of FITC conjugated (Fab)2 fragment of Rabbit antiMouse Immunoglobulins (Dako Cytomation, Ely, UK) in 0.2% BSA supplemented with 5% blocking serum for 45 minutes at 4°C in the dark. A second antibody alone control was also set up. After being washed and centrifuged as before, the resulting cell pellet is resuspended in 1ml of PBS and the percentage of positive cells ascertained using a Beckman Coulter XL Flow Cytometer (Beckman Coulter, High Wycombe, UK).

### Transplantation series 1

C57BL/6 mice (n=5) were made diabetic by injection of Streptotozocin (STZ)250mg/kg) on Day 0.750,000 PCs were injected into the tail vein on Day 3. Control animals give injection of saline or equivalent number of C57BL/6 bone marrow cells. Blood glucose was monitored every 3 days.

### Transplantation series 2

C57BL/6 mice (n=4) were made diabetic by injection of Streptotozocin (STZ 250mg/kg) on Day 0.Blood glucose

### Lipofuscin expression measurement

Cellular lipofuscin content was assessed using flowcytometry with blue emission in the region 560-590nm. Readings were calculated as mean values of the emission peaks and expressed in arbitrary units.

### WST assay methodology

Pathfinder cells were subject to oxidant insult as described below and assessed for proliferative capacity by WST assay (Roche, Switzerland) following the manufacturers instructions.

### LDH assay

LDH cytotoxicity assays were performed using a Cytotoxicity Detection Kit (Roche, Nonnenwald, Germany), following the manufacturers recommendations.

### Human Breast Tissue culture

Sample of breast tissue in 1cm pieces from patients undergoing reduction mammoplasty. Transported in 5 mls of CMRL 1066 media supplemented with 10% Foetal Bovine Serum (Sigma, Poole,UK), 2 mM Glutamax, 1.25ug/ml Amphotericin B, and 100u/ml Penicinllin/Streptomycin (all Invitrogen, Paisley). The tissue was minced under sterile conditions using scalpel blades and then treated with collagenase IV (0.05g in 10mlsHBSS) for 5-12 hours. These were syringed using a 16 guage needle and plated out in T25 tissue culture flasks in the above media at 37°C in a 5%Co2 atmosphere.

### Differentiation experiements and media

Cells were plated at 0.25 and 0.5 x106 cell per T75 and at 0.1 x 105 cells

### Hepatocyte media

DMEM :F12 ( ) supplemented with Fibroblast growth factor 4 10ng/ml (Sigma), 1 x ITS (Gibco), 100u/ml
Penicinilin/Streptomycin and 0.2% Bovine serum albumin.

### Cell Sorting

Primary antibody - mouse anti rat CD90 (Serotec)
Dynabeads Goat anti mouse IgG (Dynal Biotech)
Buffer 1: PBS (without Ca2+ and Mg2+ w/ 0.1% BSA and 2mM EDTA pH7.4.

As per maunfacturer protocol, briefly:
Dynabeads washing procedure: Transfer desired volume of resuspended dynabeads to eppendorf add same volume or at least 1ml of Buffer 1. Place tube in magnet for 1 min and discard supernatant. Remove from magnet and resuspend in original volume of buffer 1.
Add 1ug primary antibody per 10 6 target cells. Incubated for 10 minutes at 2-8°C.
Wash cells by adding 2ml Buffer 1 per 1x 107 cells and centrifuge at 300xg for 8 mins. Discard supernatant. Resuspend the cells in buffer 1 at 1 x 107 cells per ml.

### Isolation procedure:

Add dynabeads 25 ul per 1x107cells /ml of sample.
Incubate for 20 minutes at 2-8 oC with gentle tilting and rotation.
Double the volume with buffer 1 to limit trapping of unbound cells.
Place the tube in a magnet for 2 minutes.
Discard the supernatant and gently wash the bead-bound cells 4 times using following procedure: 1) Add 1 ml buffer 1per 1x107 dynabeads 2) Place tube in magnet for 1 minute and discard supernatant.
Resuspend cells in maintenance culture media and plate in tissue culture flasks.

### Depletion procedure:

Add dynabeads 50 ul per 1x107cells /ml of sample.
Incubate for 30 minutes at 2-8 oC with gentle tilting and rotation.
Double the volume with buffer 1 to limit trapping of unbound cells
Place the tube in a magnet for 2 minutes.
Transfer the supernatant containing unbound cells to a fresh tube.
Replace in magnet for further one minute.
Transfer the supernatant containing unbound cells and add to maintenance culture media and plate in tissue culture flasks.
MACS was performed on each sorted population twice before use in experiments.
All sorted population were checked with fluorescence activated flow cytometry before use in experiments.

### Senescence Associated Beta Galactosidase Assay

Cells were grown on 6-well plates at a density of 10⁵ cells per well. At approximately 72 - 96 hours after seeding (and once confluent) cells were washed in PBS, fixed for ten minutes at room temperature in 1ml 2% formaldehyde/0.2% glutaraldehyde. Rinsed twice with sterile PBS and incubated at 37°C (no CO2), and wrapped in parafilm to prevent dehydration of fresh SA β Gal staining solution, made up as follows (final concentrations are expressed): per ml of solution
- 1mg X-gal
- 5mM Potassium Ferricyanide
- 5mM Potassium Ferrocyanide
- 2mM MgCl2
- 150mM NaCl
- 40mM citric acid and sodium phosphate at pH 6 (pH4 was used as a positive control to ensure efficacy of stain in one of the six wells.)

The solution was left on the cells for up to 4 days to achieve maximum staining - ultimately microscopic analysis, showed blue precipitate within the control cells. Upon completion, staining was removed; cells washed in PBS, and stored with 3ml 70% glycerol at 4°C.

Magnification viewed the entire field for counting. The area under the eyepiece graticule was counted for both positive and negative cells. Positive, senescent cells were expressed as a percentage of the total cell count. This was repeated in six, randomly allocated, fields. A mean value for SA β Gal expression was calculated for each hydrogen peroxide treatment, from the values obtained for the six independent fields.

### RNA quantification by spectrophotometry

RNA was quantified using the GeneQuant capillary spectrophotometer (Pharmacia Biotech) following the default settings for RNA. The concentration was measured at 260 nm and the purity was estimated as the Absorbance 260/280nm ratio.

### cDNA synthesis (Reverse transcription of total RNA)

First-strand cDNA synthesis for real time PCR (Taqman) was achieved using SuperScriptTM First Strand Synthesis System (Invitrogen).

### Quantitative Real Time PCR (Taqman)

Relative Quantitative Real time PCR was used for cDNA quantification analysis in order to monitor the mRNA expression patterns of the genes of interest. The analysis was performed using the ABI Prism^{®} 7700 Sequence Detection System. Senescence associated gene expression was measured in all instances relative to a 18S RNA gene control.

The TaqManTM primers and the FAM-TAMRA-conjugated probes were designed using the Primer Express software and manufactured by Biosource UK Ltd.

### Rat RT-PCR sequences.

Primers and Probe sequences (5'-3')

| | |
|---|---|
| 18s | |
| Forward primer | acctggttgatcctgccagtag |
| Reverse Primer | agccattcgcagtttcactgtac |
| Probe: | FAM-tcaaagattaagccatgcatgtctaagtacgcac-TAMRA |

| | |
|---|---|
| XRCC5: | |
| Forward primer | tttcagcggttgtaccagtgtct |
| Reverse Primer | catattcaaaatgtgctgctgaatt |
| Probe: | FAM-ctccaggagcggctgcccc-TAMRA |

| | |
|---|---|
| p21: | |
| Forward Primer | gagccacaggcaccatgtc |
| Reverse Primer | cggcatactttgctcctgtgt |
| Probe | FAM-cctggtgatgtccgacct-TAMRA |

| | |
|---|---|
| SIRT2-sim | |
| Forward Primer | tccctcatcagcaaggca |
| Reverse Primer | gatccgtctggcctgtctt |
| Probe | FAM - tagccaccccacgactgc-TAMRA |

### STATIC INSULIN SECRETION TEST (SIST)

A static stimulation test was used as an in-vitro model to assess the ability of the islet-like clusters to secrete insulin in response to varying concentrations of glucose.

### 2.2.1 Reagent Preparation

Four stock solutions were required for the experiment: Stock Solution 1 was made by dissolving 13.44g of Sodium Chloride (ANALR BDH) in 500ml deionised water.
Stock Solution 2 was made by dissolving 4.03g Sodium bicarbonate (SIGMA), 0.75g Potassium Chloride (SIGMA) and 0.41g Magnesium chloride (SIGMA) in 500ml of deionised water.
Stock Solution 3 was made by dissolving 0.74g Calcium chloride (SIGMA) in 500ml deionised water and.
Stock Solution 4 made by dissolving 59.5g HEPES (N-2-Hydroxyethylpiperazine-N'-2-ethanesulfonic acid (SIGMA)) in 500ml deionised water and the pH corrected to pH 7.4.

The 4 stock solutions were all stored at 4°C until required to make 800ml of working solution A (KRB G0). To make KRB G0 working solution, 200ml of stock solutions 1, 2 and 3, 40 ml of stock solution 4 and 160ml of deionised water were mixed together. 800mg of bovine serum albumin (SIGMA) was added and solution adjusted to pH 7.4. Various amounts of glucose were added to KRB G0 to make 0.003M, 0.01M and 0.02M glucose solutions termed G3, G10 and G20 respectively.

### 2.2.2 Procedure

Plates removed from 37°C 5% CO2 and 95% air incubator, media discarded and washed 3 times with G3 (containing no carbachol). To induce similar levels of metabolic activity between all groups of cells, 2ml of G3 was added to all wells and the plates incubated at 37°C for 60mins under conditions of 5% CO2 and 95% air. The G3 solution was then discarded and 2ml of G3, G10 and G20 glucose concentrations were added in duplicate to appropriate wells

The plates were incubated in a 5% CO2 and 95% air incubator for 2 hours at 37°C. Thereafter, the supernatant from each well was removed and stored along with the plates at -70°C for biochemical analysis.

### RAT INSULIN ELISA

A rat Insulin ELISA kit (Mercodia) was used to determine the insulin concentrations within the supernatants. The kit is a solid phase, two-site enzyme direct sandwich immunoassay technique in which two monoclonal antibodies are directed against separate antigenic determinants on the insulin molecule. The assay was performed following the manufacturers recommmendations.

### Data analysis

The amount of insulin released into the supernatant in each well is dependent on the mass of cells present within each well. In order to standardise the insulin released within each well, the supernatant insulin concentrations were divided by the protein concentration for each individual well. The insulin concentration was then expressed as the amount of insulin released (pg) per mg of protein per minute.

### Results

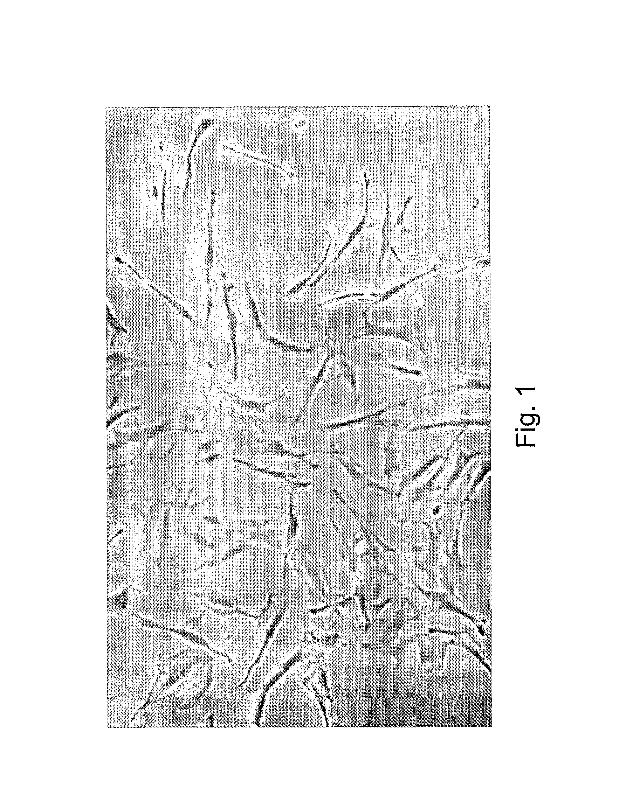

Minced ducts cultured in CMRL for five weeks resulted in the formation of a PC monolayer. The cellular morphology of these cells can be judged by the light photomicrograph shown in figure 1. These cells were subject to characterization by immuno-cytochemistry (ICC) and RT-PCR.

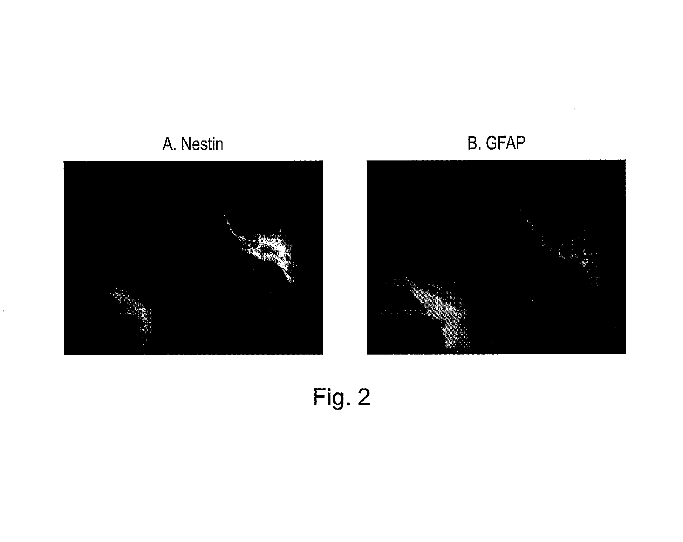

The PCs stained positive for the neural markers Nestin and GFAP by ICC (figure 2.1) and RT-PCR and for the NCAM, cMet , Oct 4 and Nestin (figure 2.2). They appeared negative for the insulin transcription factor PDX-1 and the expression of insulin, somatostatin, pancreatic polypeptide and glucagon by RT-PCR(Table 1). The PCs do not appear to express differentiated neural cell markers such as, EX2, CYCc, MBPF, AA3 and GTX, in keeping with an unspecialized status. A similar, non-identical population of cells has been described for adult mouse pancreas (27)

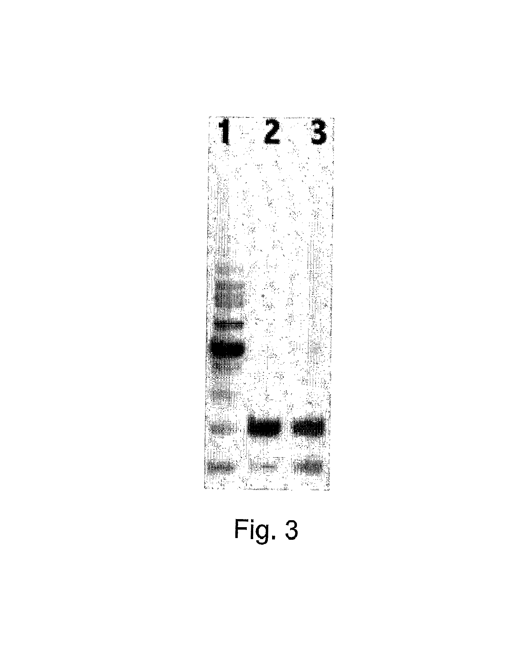

These cells and single cell isolates, have subsequently been propagated continuously in culture for 18 and 24 months without signs of growth arrest or overt change in phenotype. Telomere repeat amplification protocol (TRAP) assays have been performed on them and demonstrate that the cultures are telomerase positive (Fig 3). Traditionally, putative stem cells are thought to be telomerase negative and only reactivate telomerase during proliferation. The inventors' observation of are consistent with this view.

Previous studies on the isolation of stellate cells from the rat pancreas produced telomerase negative cultures (28)

PCs grown in culture for more than twelve months still retain the capacity to differentiate into other cell types when grown in appropriate growth medium. This again is a feature typical of stem cells.

The isolation of nestin expresing cultures that could be used to derive large numbers of cells, and facilitates the development of transplant studies previously limited by the number of cells available.

The inventors have employed administration of PCs to combat streptozotocin (STZ) induced diabetes in a rodent concordant xenograft model, with a view to tracking any functionally efficacious cells across the species barrier. C57BL/6 mice were made diabetic by injection of STZ on day 0, while 750,000 PCs were injected into the tail vein of treated animals on day 3. Control animals were give injection of saline or equivalent number of C57BL/6 bone marrow cells. Blood glucose was monitored every 3 days. The choice of autologous bone marrow (BM) as an extra control was to evaluate previous observations indicating that BM derived cells could, upon transplantation, give rise to insulin producing in STZ treated animals (4).

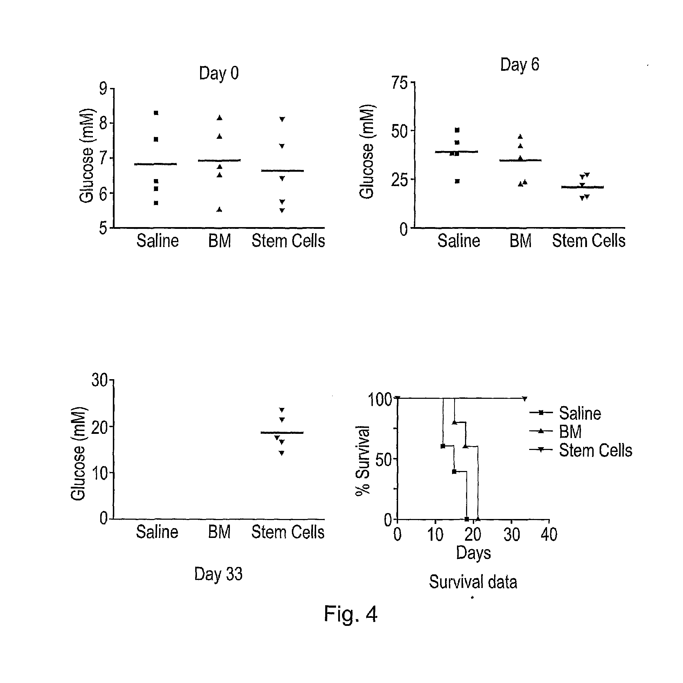

Animals treated with PCs survived STZ treatment unlike control groups (Figure 4). Following administration of STZ, blood glucose in all groups rose from a mean of 7 to 45 mM/L. This situation persisted with the saline treated controls proving non-viable by day 19, with blood glucose levels peaking at 50mM/L. The BM control group survived until day 21 before blood glucose levels precluded viability. PC treated animals survived throughout this time course and beyond to day 33, when the animals had to be sacrificed in accordance with animal license conditions The survival of the PC treated animals is highly significant (Table 2). Though normal glycaemia was not achieved, the blood glucose levels stabilised by day 6 at a mean value of 20mM/L. BM treated animals did exhibit a significant survival advantage versus saline controls (Table 2). This observation is in keeping with reports indicating bone marrow derived cells have the capacity to engender insulin production upon transplantation.

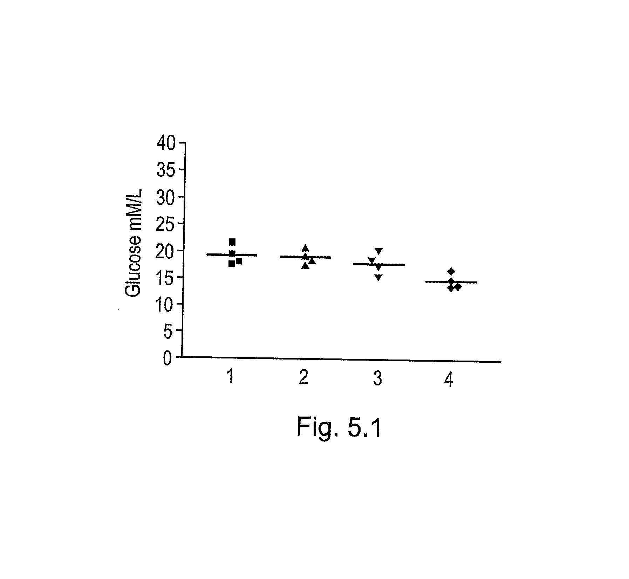

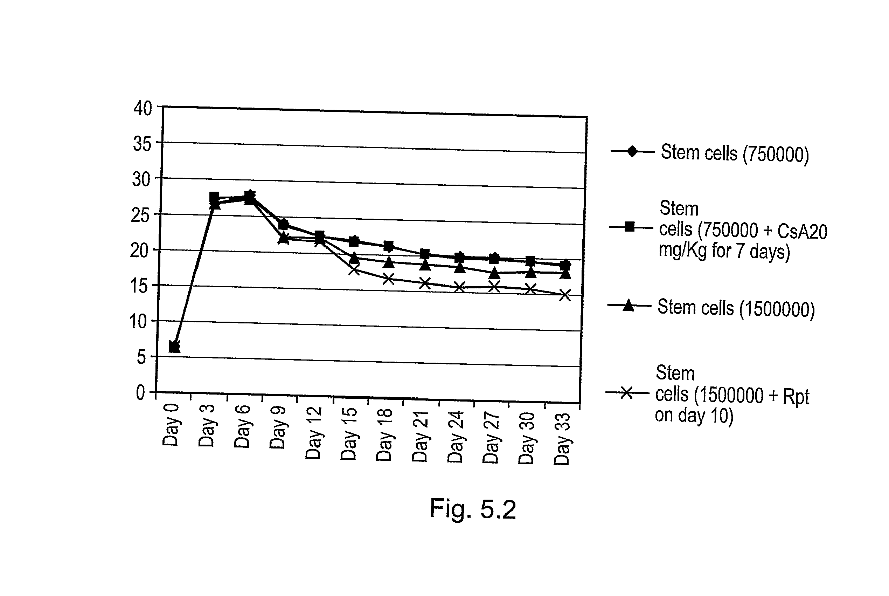

In order to establish if the lack of normolglycaemia was due to cell loss because of immune attack, or due to insufficient numbers delivered by IV, replicate transplant experiments were performed in the presence and absence of cyclosporin (CsA) treatment and with 1,500,000 cells or a repeat injection of 750,000 PCs at day 10 post STZ treatment injection. Results from this particular protocol are shown in figure 5.

CsA treatment appeared to have no effect on blood glucose levels, indicating that the PCs are non-immunogenic. This is in keeping with observations on human nestin islet precursors cells (NIPs) (12). Doubling the cell number of PCs did not significantly improve blood glucose levels to near normolglycaemia (Figure 5), with a mean of -20mM/L blood glucose being achieved, though individual animals achieved 12mM/L.

Repeat injection with 1,500,000 cells on day 10 improved the mean blood glucose level to -15mM/L by day 33 when the experiment ended. This is indicative of increasing cell number being more efficacious in mitigating the effects of STZ. An optimum cell transplant number requires to be determined. Similarly, a determination of the optimum administration timing and regime needs to be undertaken.

Post mortem analysis of the respective treated animals and control groups reveals that PC treatment regenerates the pancreatic beta cells (Figure 6). Pancreatic beta cell a structure is destroyed in STZ treated controls while it is substantially recovered in the cell transplanted mice. This has major implications for the development of any future strategies using cellular therapies to treat diabetes. The survival of these mice indicates that a substantial recovery of insulin production has been achieved. This is corroborated by ICC which indicates that the regenerated pancreas produce insulin and glucagon (Figure 7).

Significantly, the PCs appear to be non-immunogenic, which makes wide spread use of this simple IV administration of such cells to correct type 1 diabetes an attractive proposition.

RT-PCR and ICC analysis of the treated animals indicates that insulin production resulting from PC treatment is of both rat and mouse in origin. A PCR for rat Ins II (Seq ID 5) yields a common 208 bp amplicon which digests which digests with Hin fI to yield 105 bp and 103 bp fragments (Figure 8A) for rat and a 191bp and a 17bp fragment for mouse. Significantly, the embryonic form of mouse insulin is also produced (Figure 8B). RT-PCR-RFLP indicates that in PC treated animals we can detect both a mature rat insulin transcript (labeled r in both figures 8A and 8B), a mature mouse (labeled m) and an embryonic form corresponding to the Ins 1 gene product (labeled U). This has been confirmed by sequencing and cloning of the relevant fragments. The rat amplicon of 243 bp, can be digested with Hinf I to yield 140 bp and 103 bp fragments. In mouse the equivalent amplicon, generated with the same rat Ins II primers, digests to give 191bp and 52 bp bands (labeled m1 and m2 in figure 8B). STZ diabetic animals, given PCs also have a further band (labeled U in figure 8B) equivalent in size to the undigested amplicon. This corresponds to an Ins I transcript, typically observed during embryonic development. Reports of its presence in adults are equivocal.

This remains an unusual observation and provides insight into the mechanism of action of the PC cells. The presence of both transcripts indicates that the PCs have differentiated to generate insulin producing cells and have stimulated murine tissue to do likewise. Whether functionally analogus murine PCs are stimulated is undetermined. The presence of Ins I and II transcription, is suggestive of developmental reprogramming taking place in the murine tissue, though one cannot formally exclude that a population of murine ES like cells persists in the adult and is stimulated in the PC animals. No evidence for this has been observed previously. Indeed, Melton et al (30) have produced data indicating that the only source of beta cell regeneration, even after injury, is the existing beta cells and that there is no murine stem cell involvement in the generation of the mouse pancreas following damage. Our data are not fully incongruous with this observation. Indeed, they are compatible with a stimulation of undamaged tissue to serve as a substrate to regenerate the damaged tissue.

This scenario is further supported by examination of the pancreas of treated mice. Staining of pancreatic sections from these animals with polyclonal anti-rat antiserum, fails to detect any cell surface rat antigens. Control rat sections stain brightly, while control mouse and treated diabetic mice are negative for the presence of rat antigens. These observations support the observation that the endogenous murine pancreas regenerates and that this is not a consequence of widespread cell fusion with the rat Pathfinder cells.

In situ hybridization however, with rat chromosome 10 and the rat Y chromosome, does detect the presence of rat chromosomes in the pancreas of treated animals. These are rarely detected and comprise less than 1% of pancreatic islet cells in the treated animals. Their presence supports the detection of the rat insulin in the treated animals. No cells containing the rat Y chromosome and a murine signal were detected. Cells containing a triple signal for diploid mouse and rat chromosome 10, indicative of cell fusions were also detected a low levels (<1%). This is in keeping with previous reports on stem cell fusion events following transplantation.

### Example 2

### Derivation of islets from PCs

PCs can be cultured to yield islets in vitro, through growth in the appropriate medium. PC cells are differentiated by the complete removal of the CMRL medium from the flask by pipette and the addition of 10mls of HBSS (see above) for 5 minutes at room temperature to remove any excess serum. After the removal of the HBSS, 20mls DMEM/F12 medium (Invitrogen, Paisley, UK) supplemented with Penicillin/Streptomycin (as above), Amphotericin (as above ), 0.2% BSA, 10mM Niacinamide, 10ng/ml Keratinocyte Growth Factor (all Sigma, Poole, UK) and ITS-X :- (Insulin 10mg/L, Transferrin 5.5mg/L, Sodium Selenite 6.7ug/L) (Invitrogen, Paisley, UK) is added to the T75 culture flask. The cells are then maintained at 37°C in a CO2 incubator and the culture medium replaced twice weekly with 20mls of fresh medium. These produce islet clusters (Fig 8.1) or neurones, in the presence or absence of serum.

Islet clusters, derived as described above, stain positively for the presence of insulin (Figure 8.2A) and contain dense core vesicles with an electron translucent halo, typical of insulin containing vesicles (Figure 8.2B). The clusters are positive for the presence of insulin, somatostatin, pancreatic polypeptide and glucagon, as judged by RT-PCR analysis. Critically, they also express Pdx-1, as judged by RT-PCR (Table 1) and Insulin and glucagons (Figure 10). Sequence identities are listed in the appendix.

The PCs can be sorted by FACs, using the rat marker Thy-1, to yield two distinct populations based on the presence or absence of expression of this marker (Figure 11)

Prolonged growth in culture results in the accumulation of Senescence associated beta galactosidase (SA β.Gal), indicative of the onset of senescence in the culture. This typically reflects an accumulation of cell damage. This is a novel finding, as ES cells are typically refractory to the accumulation of this marker with prolonged culture (29). No such accumulation with increasing passage was observed with an established marker of oxidant damage, lipofuscin. This has implications in the filed, relative to the elucidation of putative cancer stem cells and the safety of damaged cells when used in transplant experiments or in other regenerative medicine strategies, such as breast reconstruction.

### Example 3

### Pathfinder cells exhibit stem cell characteristics, but show differences in their response to oxidative insult.

### Growth characteristics of PCs

Pathfinder cells demonstrate exponential growth and exhibit no increase in population doubling times and the absence of a growth plateau. (Figure 12).

This is consistent with observations on ES cells, which indicate that these cells can remain undifferentiated in vitro for extended periods Figure 1 illustrates two individual cultures of rat PCs (A and B) passaged sequentially in culture for 172 days, without senescing. The total number of cells obtained from initial isolation to the 172 day time point is in the order of 1 x 10³⁴. This illustrates the utility of these cells in producing suitable numbers of cells for transplantation.

### Determination of Lipofuscin content in serially passaged PCs

Lipofuscin is a non degradable, auto-fluorescing pigment that accumulates progressively with age in the lysosomes of replicating cells. Its accumulation is reported to be accelerated under conditions of increased oxidative insult. Larger cells, with elevated lipofuscin content are typically senescent (Figure 13. Mean lipofuscin content in the Pathfinder population demonstrated a reduction with serial passage. However, when a FACs gating protocol was used selecting a consistent population of cells by size and granularity there was no significant change in lipofuscin content. These data demonstrate that there is an apparent sub-population of PCs that have a high Lipofuscin content, and exhibit the size characteristics of senescent cells. This sub-population is lost with serial passage , as the remaining cells show no change in lipofuscin content over time in culture.

### Proliferation of PCs in the presence of elevated oxidant stress levels

Unlike ES cells (31) Pathfinder cells do show a sensitivity to oxidative insult as determined by WST assay. The WST assay is based on the cleavage of the tetrazolium salt WST-1 to formazan by cellular mitochondrial dehydrogenases. Expansion in the number of viable cells results in an increase in the overall activity of the mitochondrial dehydrogenases in the sample. The augmentation in enzyme activity leads to the increase in the amount of formazan dye formed. The formazan dye produced by viable cells was quantified by a multi-well spectrophotometer by measuring the absorbance of the dye solution at 440 nm.

Data from these analyses are shown in Figure 14. A concentration of 100 µM is sufficient to reduce PC proliferation by 50%.

### SA β Gal analyses

SA β Gal is a classical biomarker for oxidant stress in mammalian cells, which accumulates with increasing oxidative damage. It has been proposed as a biomarker for biological ageing and has been observed to increase in human tissues with increasing chronological age. The inventors have observed an increase in SA β Gal expression in PCs with increasing passage in culture. (Figure 15). This percentage of SA β Gal expression reduced significantly with extended serial passage (Figure 15). Furthermore, from passage 16 onward the percentage of cells expressing SA β Gal has remained below 10%. (Figure 15). This is a feature typical of non-senescent cells or those with superior anti-oxidant defences.

Conversely, Pathfinder cells do exhibit sensitivity to acute oxidant stress. A significant increase in SA β Gal expression was observed with increasing concentrations of hydrogen peroxide.(Figure 16). This is in keeping with the observations following oxidant challenge in the WST assay.

The data obtained by the inventors indicates that Pathfinder cells, unlike typical stem cells, show a differential sensitivity to oxidant stress. Unlike MSCs they do not appear to show replicative senescence and can be grown for prolonged periods of time in culture (years). Consequently, lipofusicin and SA β.Gal expression decreases in later passages of these cells, Lipofuscin analysis indicates that early passages of Pathfinders contain a sub-population of cells that show features of are senescence (Figure 13). This is supported by SA β Gal analysis, with evidence that only a small proportion (<10%) of later passage cells express SA β Gal . This is congruent with a sub population of the initial Pathfinder isolate undergoing senescence, while surviving cells grow out. The resultant population of cells develops an oxidant sensitive phenotype, unlike ES cells.

Again, unlike ES cells, this situation can be reversed following oxidant insult with hydrogen peroxide. Cells challenged with a mild concentration of hydrogen peroxide (25µM) exhibit an elevation in SA β Gal expression. Furthermore, the level of SA β Gal expression is elevated relative to the time the cells have spent in culture. This is illustrated in (Figure 17).

PCs showed a dose dependant elevation in SA β Gal expression in response to challenge with either 11.25 or 25µM H₂O₂. Two passages (NP 27 and NP 28) corresponding to a difference of 8 cell divisions, had significantly higher SA-β-Gal expression (p<0.001) (Figure 17), following the sub-lethal oxidant challenge (as determined by the WST assay). Despite the cells being subject to a mild oxidant challenge, they still showed substantial oxidant sensitivity, with up to 40% of cells showing SA β Gal expression.

### LDH cytotoxicity Assay

The LDH Assay provides a method for quantitating cytotoxicity based on the measurement of activity of lactate dehydrogenase (LDH) released from damaged cells. LDH is an oxidoreductase that catalyzes the interconversion of lactate and pyruvate. It is stable and is a cytoplasmic enzyme present in all cells. Damage to cell plasma membrane results in the rapid release of LDH into the cell culture supernatant. The assay is based on the reduction of the tetrazolium salt INT in a NADH-coupled enzymatic reaction to formazan, which is water-soluble and exhibits an absorption maximum at 492 nm. The intensity of the red colour formed is increased in the presence of increased LDH activity. Figure 18 shows the mean level of LDH activity in PCs following challenge by increasing concentrations of H₂O₂ over the range 0-300µM.

The data (Figure 18) indicate that H₂O₂ challenge results in cytotoxic effects, even at low levels, with a concentration of 25µM resulting in near 30% toxicity. These observations are supportive of the WST and SA β Gal data, illustrating the sensitive nature of these cells to acute oxidant challenge, unlike stem cells. The apparent reduction in LDH activity at higher H₂O₂ concentrations is reflective of cell loss in these cultures.

### Senescence associated gene analysis in PCs under acute oxidant stress

Molecular analyses of PCs treated with 25µM H₂O₂ provides supportive data for the WST, LDH , SA β Gal and Lipofuscin studies.

The data provided by the inventors indicates that Pathfinder cells, unlike typical stem cells, show a differential sensitivity to oxidant stress. Unlike MSCs they do not appear to show replicative senescence and can be grown for prolonged periods of time in culture (years). However, lipofusicin and SA β Gal expression are increased in Pathfinder cells following oxidant insult with hydrogen peroxide. Despite the cells being subject to a mild oxidant challenge, as determined by WST assay, they still showed substantial oxidant sensitivity, with ~30% of cells showing SA β Gal expression.

These observations were supported by senescence associated gene expression. Quantitative real time PCR (qRT-PCR) was used to estimate mRNA levels corresponding to the candidate senescence associated genes. These comprised p21 as a marker of acute cellular damage responses; SIRT 2 as a regulator of cell cycle and XRCC5 as a marker of DNA damage. Assessment of relative stress and senescence associated gene expression demonstrated a statistically significant decrease (p<0.001) in all three genes following treatment with H2O2 (Figure 19). This is suggestive of the onset of a p53 damage response and ultimate priming for apoptosis following oxidative damage. Activation of P21 facilitates phosphorylation of pRb by the cyclin A-Cdk2,cyclin E-Cdk2 and cyclin D1-cdk4 complexes and has been found to be over expressed in replicative senescent fibroblasts and in cells that have undergone oxidative stress-induced premature senescence. The observed decrease in XRCC5 is consistent with stress induced senescence, as its product has been shown to be downregulated in damaged or senescent cells following acute insult. and Sirtuin 2 (SIRT 2) is a member of the silent information regulator gene family and are involved in essential cell processes, linking telomere biology with mitochondrial function and ribosome production. SIRT2 is a cell cycle regulator responsive to intracellular redox changes. It has been shown to interact with and deacetylate the a-tubulin subunit of microtubules and is thought to participate in a late mitotic check-point to ensure correct chromosome segregation during cytokinesis.

### Example 4

PCs have been identified and characterised based on the expression of Nestin (as determined by RT-PCR) and a number of other markers (Tables 3 and 4). The Nestin expression status of the Pathfinder population is mixed, however, when assessed by flowcytometry, with up to 50% of the cells being Nestin negative. The marker profiles below are provided to be taken in context of the presence or the absence of Nestin and the other markers documented in Table 1.

The unsorted PCs can be sub-divided into distinct subpopulations based on the expression of cell surface markers. The composition of this cell surface marker panel varies with time spent in culture.

Early passages of unsorted cell express the following marker profile, as illustrated by passage 4 cells:

| | |
|---|---|
| CD 90 | ~50% + |
| CD49f | ~90% + |
| CD24 | low |
| CD147 | ~90%+ |
| CD45 | Negative |
| CD44 | low |
| CD71 | Negative |
| CD31 | Negative |
| CD117 | (a.k.a c-Kit) Negative |
| Vimentin | v. low |
| ABCG2 | Negative |
| CK 19 | Negative |

| | |
|---|---|
| Low = ~less or equal to 5% of cells express marker v. Low = ~less or equal to 2.5% express marker | |

Later passages of the same population express the following profile, as illustrated by passage 28 cells (-200days in culture, 200 population doublings).

| | |
|---|---|
| CD90 | ~15%+ |
| CD49f | ~95%+ |
| CD24 | ~60%+ |
| CD147 | ~90%+ |
| CD45 | Negative |
| CD44 | low |
| CD71 | low |
| CD31 | Negative |
| CD 117 | Negative |
| ABCG2 | low |
| CK 19 | v low |

Individual passages can be sorted into CD 90 + and CD 90 - populations by FACs. These appear morphologically distinct under light microscopy

### Example 5

Cells isolated by serial dilution from the unsorted population can be used to produce a number of other cell types including neural progenitor cells. This is surprising for a cell derived from adult pancreas
This is illustrated in Figure 20 showing the production of a pure cell population of neural progenitor cells.

### Example 6

Pathfinder cells can be grown in hepatocyte differentiation media. Both CD90 + and CD90 - cells exhibit a morphology change when grown in defined hepatocyte (Figure 21) differentiation media (see Materials and Methods) and express the hepatobiliary marker Cytokeratin 19, as determined by RT-PCR. The morphology of the CD90- cells grown in differentiation medium is typically hepatic.

The CD 90- cell population expresses the following cell surface marker profile:

| | |
|---|---|
| CD90 | Negative |
| CD49f | ~95% + |
| CD24 | ~80%+ |
| CD147 | ~80%+ |
| CD45 | Negative |
| CD44 | ~60%+ |
| CD71 | low |
| CD31 | Negative |

The CD90 + cells express the following cell surface marker profile:

| | |
|---|---|
| CD90 | + |
| CD49f | ~95% + |
| CD24 | Negative |
| CD147 | ~90%+ |
| CD45 | Negative |
| CD44 | ~85%+ |
| CD71 | low |
| CD31 | Negative |
| C-KIT | Negative |

Both these marker profiles are considered to be expressed with all possible combinations of Nestin and the markers listed in Table 1.

Growth of sorted cell populations in defined media (see Example 2) to produce islets indicates that only CD90+ cells have the capacity to produce islet structures. This is illustrated in Figure 22, where islet clumps are shown in panel B, develop from PCs.

CD90 + cells accumulate to produce islet structures in defined growth medium, while CD 90- cells remain unchanged when viewed under the light microscope.

### Example 7

A number of human tissues have been processed to isolate PCs by the same general methodology as employed for the processing of rat pancreas (see Materials and Methods). This can be exemplified by the isolation of cells from the adult human breast.

Human PCs have also been isolated from minced human cortical kidney biopsies, using the same general methodology. The breast PCs are illustrated in Figure 23.

### Example 8

PCs can be differentiated into islets when grown in appropriate differentiation media (see Materials and Methods). The islets produced release insulin when stimulated to do so in vitro by exposure to glucose. Figure 24 illustrates the insulin release profile for such islets in the presence of increasing concentrations of glucose (3-20mM). The data illustrate a statistically greater level of insulin release (p<0.001) from the islets compared to undifferentiated cells.

### Deposit of Material

The following material have been deposited with The European Collection of Cell Cultures, Porton Down, Salisbury, Wiltshire, UK, SP4 OJG:

| Material | ECACC No. | Deposit Date |
|---|---|---|
| Adult rat PCs 2005 | Q6203 | 12 May |

This deposit was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ECACC under the terms of the Budapest Treaty, and subject to an agreement between The University Court of the University of Glasgow and ECACC, which assures permanent and unrestricted availability of the biological material of the deposit to the public upon issuance of the pertinent patent or upon laying open to the public of any patent application, whichever comes first, and assures availability of the biological material to one determined by the respective Patent office to be entitled thereto according to R. 28 (4) EPC or the equivalent in other jurisdictions.

In this regard the applicant hereby declares under R. 28 (4) EPC, or equivalent rule in other jurisdictions, that availability of the biological material referred to above and herein shall be effected only by the issue of a sample to an expert.

The applicant of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

Appendix

Sequence Ids
Seq. ID 1
A. Nestin forward primer 5′-CTAGAGCTGTTTCCAGGTGCCA-3′
B. Nestin reverse primer 5′-CCAGCCATTTCCTTACCTGT-3′

Seq. ID 2
A. NCAM forward primer 5′-CAGCGTTGGAGAGTCCAAAT-3′
B. NCAM reverse primer 5′-TTAAACTCCTGTGGGGTTGG-3′

Seq. ID 3
A.cMET forward primer 5′-CATTCTGCTGCTGCTGCTGA-3′
B.cMET reverse primer 5′-TCACTACACAGTCGGGACAC-3′

Seq. ID 4
A. GFAP forward primer 5′- CCCTGTCTCGAATGACTCCTCCA-3′
B. GFAP reverse primer 5′- GGAGTTCTCGAACTTCCTCCTCA-3′

Seq ID 5
A.Ins II forward primer 5′-ATGGCCCTGTGGATCCGCTT-3′
B.Ins II reverse primer 5′-TGCCAAGGTCTGAAGGTCAC-3′
C. Ins II nested primer 5′-5′-CCTGCTCATCCTCTGGGAGCC-3′

Seq ID 6
A. Ins II forward primer 5′-
B. Ins II reverse primer 5′-

Seq ID 7
A. Somatostatin forward primer 5′-CTAGAGCTGTTTCCAGGTGCCA-3′
B. Somatostatin reverse primer 5′-CCCAGCCATTTCCTTACCTGT-3′

Seq ID 8
A Pancreatic polypeptide forward 5′AGTCCAGCAGAAGGTAGGTGTC-3′
B Pancreatic polypeptide reverse 5′-AGTTGTTGCAAGAGCTGGGC-3′

Seq ID 9
A Glucagon forward primer 5′-GACCGTTTACGTGGCTGG-3′
B Glucagon reverse primer 5′-CGGTTCCTCTTGGTGTTCATCAAC-3′

Seq ID 10
PDX-1 forward primer 5′-ATCACTGGAGCAGGGAAGGT-3′
PDX-1 reverse primer 5′-GCTACTACGTTTCTTATCT-3′

Seq ID 11
CycC Forward 5/ -ACCCCACCGTGTTCTTCGAC-3/
CycG Reverse 5/-CATTTGCCATGGACAAGATG-3/

Seq ID 12
Ex2 Forward 5/-GCTCTCACTGGTACAGAA-3/
Ex2 Reverse 5/-TACATTCTGGCATCAGCGCAGAGACTGC-3/

Seq ID 13
Mbp Forward 5/-ATAACCATTCCCTGCCTC-3/
Mbp Reverse 5/-CTTTTTCTCTTACCCCCAC-3/

Seq ID 14
Mobp Forward 5/-ACAGACAGTATTACGGTGGC-3/
Mobp Reverse 5/-GTTACCGTAGTCGAGAAGGA-3/

Seq ID 15
Gtx Forward 5/-GCTCCTGGATAAGGATGGCAA-3/
Gtx Reverse 5/-CTTTTTGAGAAGCCGCGTGA-3/

Seq ID 16
Myt1 Forward (5/-GCGAGACCAACCCACAGGACA-3/
Myt1 Reverse (5/-TTACGTGGCCGGTTCCATCACA-3/

**TABLE 1**

| **Marker expressed by PCs** | | |
|---|---|---|
| **Marker** | **PCs** | **Islet like clusters** |
| | | |
| **Nestin** | **+** | - |
| **cMet** | **+** | **+** |
| **NCAM** | **+** | - |
| **GFAP** | **+** | - |
| **EX2** | - | - |
| **NF68** | - | - |
| **CYCc** | **+** | - |
| **MBFP** | - | - |
| **GTX** | - | - |
| **PDX-1** | - | **+** |
| **Insulin** | - | **+** |
| **Glucagon** | - | **+** |
| **Somatostatin** | - | **+** |
| **PP** | - | **+** |

**TABLE 2**

| | | |
|---|---|---|
| **Statistical analysis of survival data** | | |

| **Group** | **Mean Survival Time (N=6)** | **P Value** |
|---|---|---|
| **Saline** | **15.0±2.683** | |
| **Bone Marrow** | **19.2±2.4** | **0.0439*** |
| **Stem Cells** | **>30** | **0.0025*** |
| | | **0.0034#** |

| | | |
|---|---|---|
| * **Compared to saline, # Compared to BM** | | |

**TABLE 3**

| **Markers for PC characterisation** |
|---|
| Nestin Nucleostemmin Sox 1ABCG2 CD133 CXCR4 FABP7 Glut 1 Frizzled 9 Musashi 1,2 |
| Oct-4, Oct 3, Nanog,ABCG2 Alkaline Phosphatase STAT3 SOX 2 SSEA 1PODXL |
| Integrin alpha 6, beta1 CD9,24,133, CCR4 |
| Integrin alpha 1 Sca1 Stro 1 VCAM 1 Nucleostemmin c-Kit BMP-R5 |

| **Early passages of unsorted cell express the following marker profile, as illustrated by passage 4 cells:** | |
|---|---|
| | |
| CD 90 | ~50% + |
| CD49f | ~90% + |
| CD24 | low |
| CD147 | ~90%+ |
| CD45 | Negative |
| CD44 | low |
| CD71 | Negative |
| CD31 | Negative |
| CD117 | (a.k.a c-Kit)Negative |
| Vimentin | v. low |
| ABCG2 | Negative |
| CK 19 | Negative |

| | |
|---|---|
| Low = ~less or equal to 5% of cells express marker V. Low = ~less or equal to 2.5% express marker | |

| **Later passages of the same population express the following profile, as illustrated by passage 28 cells (~200days in culture, 200 population doublings).** | |
|---|---|
| | |
| CD90 | ~15%+ |
| CD49f | ~95% + |
| CD24 | ~60%+ |
| CD147 | ~90%+ |
| CD45 | Negative |
| CD44 | low |
| CD71 | low |
| CD31 | Negative |
| CD 117 | Negative |
| ABCG2 | low |
| CK 19 | v low |

**TABLE 3 CONT'D.**

| **The CD 90- cell population expresses the following cell surface marker profile:** | |
|---|---|
| | |
| CD90 | Negative |
| CD49f | ∼95% + |
| CD24 | ∼80%+ |
| CD147 | ∼80%+ |
| CD45 | Negative |
| CD44 | ∼60%+ |
| CD71 | low |
| CD31 | Negative |

| **The CD90 + cells express the following cell surface marker profile:** | |
|---|---|
| | |
| CD90 | + |
| CD49f | ∼95% + |
| CD24 | Negative |
| CD147 | ∼90%+ |
| CD45 | Negative |
| CD44 | ∼85%+ |
| CD71 | low |
| CD31 | Negative |
| C-KIT | Negative |

**TABLE 4**

| **Examples of Senescence and damage markers for use in assessing PCs** |
|---|
| |
| Rif1, Rif2, Rap1, |
| SIRTs1,2,3,4 ,56,7,; Est1,Est2;TLG1,cdc13,A26,ATM,HDAC1,hSEP1,hTEP1,Hu |
| Cds1,MYC, |
| NEK2,p21,PIN2,TNKS,TERC,hTERT,TOP2A,TOP2B,TP53,TRF1,TRF2,WRN, |
| G22P1, XRCC5 ,POT1, Collagenase, caveolin-1,p16,p21,gamma |
| H2AX,Chk1,Chk2,Rad 17,BRCA1 MRE11, ATR, ATRIP. |

### References

1. Eur J Endocrinol. 2004 Nov;151 Suppl 3:U7-12.
   Stem cell research: immortality or a healthy old age? Mummery C.
2. J Clin Invest. 2004 Nov;114(9):1184-6.
   Embryonic death and the creation of human embryonic stem cells.
   Landry DW, Zucker HA.
3. http://stemcells.nih.gov/info/scireport/
4. Colman A
   Making new beta cells from stem cells.
   Semin Cell Dev Biol. 2004 Jun;15(3):337-45.
5. Jackson, K., Majka SM, Wang H, Pocius J, Hartley CJ, Majesky MW, Entman ML, Michael LH, Hirschi KK, and Goodell MA (2001). Regeneration of ischemic cardiac muscle and vascular endothelium by adult stem cells. J. Clin. Invest. 107, 1-8
6. Johe, K.K., Hazel, T.G., Muller, T., Dugich-Djordjevic, M.M., and McKay, R.D. (1996). Single factors direct the differentiation of stem cells from the fetal and adult central nervous system. Genes Dev. 10, 3129-3140.
7. Krause, D.S., Theise, N.D., Collector, M.I., Henegariu, O., Hwang, S., Gardner, R., Neutzel, S., and Sharkis, S.J. (2001). Multi-organ, multi-lineage engraftment by a single bone marrow-derived stem cell. Cell. 105, 369-377.
8. Bianco, P. and Cossu, G. (1999). Uno, nessuno e centomila: searching for the identity of mesodermal progenitors. Exp. Cell Res. 251, 257-263
9. Anderson, D.J., Gage, F.H., and Weissman, I.L. (2001). Can stem cells cross lineage boundaries? Nat. Med. 7, 393-395.
10. Lagasse, E., Connors, H., Al Dhalimy, M., Reitsma, M., Dohse, M., Osborne, L., Wang, X., Finegold, M., Weissman, I.L., and Grompe, M. (2000). Purified hematopoietic stem cells can differentiate into hepatocytes in vivo. Nat. Med. 6, 1229-1234.
11. Kocher, A.A., Schuster, M.D., Szabolcs, M.J., Takuma, S., Burkhoff, D., Wang, J., Homma, S., Edwards, N.M., and Itescu, S. (2001). Neovascularization of ischemic myocardium by human bone-marrow-derived angioblasts prevents cardiomyocyte apoptosis, reduces remodeling and improves cardiac function. Nat. Med. 7, 430-436.
12. Zulewski H, Abraham EJ, Gerlach MJ, Daniel PB, Moritz W, Muller B, Vallejo M, Thomas MK, Habener JF: Multi-potential nestin-positive stem cells isolated from adult pancreatic islets differentiate ex vivo into pancreatic endocrine, exocrine, and hepatic phenotypes. Diabetes 50:521-533, 2001
13. Serup P, Madsen OD, Mandrup-Poulsen T: Science, medicine, and the future: islet and stem cell transplantation for treating diabetes. BMJ 322:29 -32, 2001
14. Vinik A, Pittenger G, Rafaeloff R, Rosenberg L, Duguid WP: Determinants of pancreatic islet cell mass: a balance between neogenesis and senescence/ apoptosis. Diabet Rev 4:235-263, 1996
15. Bonner-Weir S, Baxter LA, Schuppin GT, Smith FE: A second pathway for regeneration of adult exocrine and endocrine pancreas: a possible recapitulation of embryonic development. Diabetes 42:1715-1720, 1993
16. Gu D, Sarvetnick N: Epithelial cell proliferation and islet neogenesis in IFN-g transgenic mice. Development 118:33- 46, 1993
17. Cornelius JG, Tchernev V, Kao KJ, Peck AB: In vitro generation of islets in long-term cultures of pluripotent stem cells from adult mouse pancreas. Horm Metab Res 29:271-277, 1997
18. Ramiya VK, Maraist M, Arfors KE, Schatz DA, Peck AB, Cornelius JG:
   Reversal of insulin-dependent diabetes using islets generated in vitro from
      pancreatic stem cells. Nat Med 6:278 -282, 2000
19. Bonner-Weir S, Taneja M, Weir GC, Tatarkiewicz K, Song KH, Sharma A, O'Neil JJ: In vitro cultivation of human islets from expanded ductal tissue. Proc Natl Acad Sci U S A 97:7999-8004, 2000
20. Hunziker E, Stein M: Nestin-expressing cells in the pancreatic islets of Langerhans. Biochem Biophys Res Commun 271:116 -119, 2000;
21. Lumelsky N, Blondel O, Laeng P, Velasco I, Ravin R, McKay R: Differentiation of embryonic stem cells to insulin-secreting structures similar to pancreatic islets. Science 292:1389 -1394, 2001
22. Piper K, Ball SG, Turnpenny LW, Brickwood S, Wilson DI, Hanley NA:Beta-cell differentiation during human development does not rely on nestin-positive precursors:
   implications for stem cell-derived replacement.
   Diabetologia. 2002 Jul;45(7):1045-7
23. Jiang FX, Cram DS, DeAizpurua HJ, Harrison LC: Laminin-1 promotes differentiation of fetal mouse pancreatic beta-cells. Diabetes 48:722-730, 1999
24. Crisera CA, Kadison AS, Breslow GD, Maldonado TS, Longaker MT, Gittes GK: Expression and role of laminin-1 in mouse pancreatic organogenesis.Diabetes 49:936 -944, 2000
25. Otonkoski T, Beattie GM, Mally MI, Ricordi C, Hayek A:
   Nicotinamide is a
      potent inducer of endocrine differentiation in cultured human fetal pancreatic cells. J Clin Invest 92:1459 -1466, 1993
26. Ru Gao, Jarkko Ustinov, Mari-Anne Pulkkinen, Karolina Lundin, Olle Korsgren and Timo Otonkoski Adult Pancreatic Cell Culture. Diabetes 52:2007-2015, 2003
27. Seaberg RM, Smukler SR, Kieffer TJ, Enikolopov G, Asghar Z, Wheeler MB, Korbutt G, van der Kooy D. Clonal identification of multipotent precursors from adult mouse pancreas that generate neural and pancreatic lineages. Nat Biotechnol. 2004 Sep;22(9):1115-24.
28. Masamune A, Satoh M, Kikuta K, Suzuki N, Shimosegawa T. Establishment and characterization of a rat pancreatic stellate cell line by spontaneous immortalization. World J Gastroenterol. 2003 Dec;9(12):2751-8.
29. Saretzki G, Armstrong L, Leake A, Lako M, von Zglinicki T. Stress defense in murine embryonic stem cells is superior to that of various differentiated murine cells. Stem Cells. 2004;22 (6) :962-71
30. Dor Y, Brown J, Martinez OI, Melton DA. Adult pancreatic beta-cells are formed by self-duplication rather than stem-cell differentiation. Nature. 2004 May 6;429(6987):41-6.
31. Armstrong L, Saretzki G, Peters H, Wappler I, Evans J, Hole N, von Zglinicki T, Lako M. Overexpression of telomerase confers growth advantage, stress resistance, and enhanced differentiation of ESCs toward the hematopoietic lineage.Stem Cells. 2005 Apr;23(4):516-29
   1. An isolated cell population originating from adult tissue, wherein said cells are Nestin positive and capable of growth in a Matrigel free culture system in the presence of serum.
   2. An isolated cell population according to paragraph 1 wherein said cells are PDX-1 negative.
   3. An isolated cell population according to paragraph 1 or paragraph 2 wherein the adult tissue is pancreatic, bone marrow, heart, breast, liver or kidney tissue.
   4. An isolated cell population according to paragraph 3 wherein the adult tissue is human.
   5. An isolated cell population according to paragraph 4 wherein the adult tissue is pancreatic.
   6. An isolated cell population according to paragraph 5 comprising cells deposited under accession number Q6203 at ECACC on 12 May 2005.
   7. A pharmaceutical composition comprising an isolated cell population in accordance with any one of the preceding paragraphs along with a pharmaceutically acceptable carrier.
   8. A method of isolating a multipotent stem cell population from adult mammalian tissue, said method comprising
      culturing said adult mammalian tissue, and
      isolating emergent cell population monolayer.
   9. A method according to paragraph 8 wherein the adult mammalian tissue is selected from the group consisting of pancreas, breast, liver and kidney.
   10. A method according to paragraph 8 or paragraph 9 wherein the tissue is human.
   11. A method according to any one of paragraphs 8 to 10 wherein the cell population is in accordance with any one of paragraphs 1 to 6.
   12. A method according to any one of paragraphs 8 to 11 wherein the tissue is cultured in CMRL-1066 medium (Sigma - C-0422).
   13. A method according to any one of paragraphs 8 to 12 wherein the tissue is from adult pancreatic ducts comprising whole ducts.
   14. A method according to paragraph 13 wherein the duct tissue is minced to aid in the culturing step.
   15. An adult cell population producible by a method according to any one of paragraphs 8 to 14.
   16. A method of treating a disease state associated with cell degeneration or age related tissue change, said method comprising the steps of administering a cell population according to any one of paragraphs 1 to 6 or a pharmaceutical composition according to paragraph 7 to a patient having said disease or age related condition.
   17. A method according to paragraph 16 wherein the cells are administered intravenously to said patient.
   18. A method according to paragraph 16 wherein the cells are transplanted to the disease site or site of age related degeneration.
   19. A method according to any one of paragraphs 16 to 18 wherein the disease is associated with degeneration of pancreatic cells, neuronal cells, cardiovascular cells, epithelial cells, liver cells, muscle cells, retinal, cells, hair follicle or kidney cells.
   20. A method according to paragraph 19 wherein the disease is diabetes (type I and II), Parkinson's disease, Alzheimer's disease kidney disease, eye disease, liver disease or cardiovascular disease.
   21. A method according to any one of paragraphs 16 to 18 wherein the donor of the cells and the patient are the same species.
   22. A method according to paragraph 21 wherein the patient is human.
   23. A method according to any one of paragraphs 14 to 18 wherein the donor of the cells is rat and the patient is human.
   24. A method of producing a specified differentiated cell population, said method comprising the steps of
      providing a PC population;
      optionally, selecting a PC cell subpopulation using one or more markers provided in Table 3; and
      culturing said subpopulation of cells in an environment conducive to cell differentiation.
   25. A method according to paragraph 24 further comprising the step of determining suitability for transplantation of given PC cells and derived populations of differentiated and undifferentiated cells for transplantation comprising
      detecting the presence in the cell population of one or more of the markers provided in Table 4.
   27. An isolated stem cell population according to any one of paragraphs 1 to 6 for use in a method of medical or cosmetic treatment.
   28. Use of an isolated stem cell population according to any one of paragraphs 1 to 6 in the preparation of a medicament for treating a disease state associated with cell degeneration.
   29. Use according to paragraph 27 wherein the disease is diabetes, liver disease, kidney disease, eye disease, cardiovascular disease or Parkinson's disease.
   30. A method of cosmetic tissue enhancement, said method comprising the steps of administering a cell population according to any one of paragraphs 1 to 6 or a pharmaceutical composition according to paragraph 7 to a patient requiring such enhancement.
   31. A method according to paragraph 30 wherein the cosmetic tissue enhancement is treating a state associated with cell degeneration or age related tissue change.
   32. A method according to paragraph 30 or 31 wherein the tissue enhancement is limb or organ regrowth or replacement.
   33. A method according to paragraph 31 or paragraph 32 wherein the cells are administered intraveneously to said patient.
   34. A method according to paragraph 31 or paragraph 32 wherein the cells are transplanted to the site of cell or age related degeneration or site of insult.
   35. A method according to any one of paragraphs 30 to 34 wherein the donor of the cells and the patient are the same species.
   36. A method according to paragraph 35 wherein the patient is human.
   37. A method according to any one of paragraphs 30 to 34 wherein the donor of the cells is rat and the patient is human.
   38. Use of an isolated cell population according to any one of paragraphs 1 to 6 in the preparation of a treatment for cosmetic tissue enhancement.
   39. A method of obtaining secreted cellular factors of a cell population according to any one of paragraphs 1 to 6 comprising culturing said cell population in suitable media and isolating said factors from said media.
   40. Use of a cellular factor obtained from a cell population according to any one of paragraphs 1 to 6 in the preparation of a medicament for treating a disease state associated with cell degeneration.
   41. Use of a cellular factor obtained from a cell population according to any one of paragraphs 1 to 6 in the preparation of a medicament for treating a disease state associated with cell degeneration.

## Claims

1. An isolated cell population originating from adult tissue, optionally adult human tissue, wherein said cells are Nestin positive and capable of growth in a Matrigel free culture system in the presence of serum.

2. An isolated cell population according to claim 1 wherein: (a) said cells are PDX-1 negative; and/or (b) he adult tissue is pancreatic, bone marrow, heart, breast, liver or kidney tissue; and/or (c) the population comprises cells deposited under accession number Q6203 at ECACC on 12 May 2005.

3. A pharmaceutical composition comprising an isolated cell population in accordance with claim 1 or claim 2 along with a pharmaceutically acceptable carrier.

4. A method of isolating a multipotent stem cell population from adult mammalian tissue, optionally adult human tissue, said method comprising culturing said adult mammalian tissue; and isolating an emergent cell population monolayer.

5. A method according to claim 4 wherein: (a) the adult mammalian tissue is selected from the group consisting of pancreas, breast, liver and kidney; and/or (b) the cell population is in accordance with claim 1 or claim 2; and/or (c) the tissue is cultured in CMRL-1066 medium (Sigma - C-0422); and/or (d) the tissue is from adult pancreatic ducts comprising whole ducts, optionally wherein the duct tissue is minced to aid in the culturing step.

6. An adult cell population producible by a method according to claim 4 or claim 5.

7. A method of obtaining secreted cellular factors of a cell population according to claim 1 or claim 2 comprising culturing said cell population in suitable media and isolating said factors from said media.

8. A cell population according to claim 1 or claim 2, pharmaceutical composition according to claim 3 or cellular factor obtained from a cell population according to claim 1 or claim 2, for use in a method of medical or cosmetic treatment, for example in a method of treating a disease state associated with cell degeneration or age related tissue change.

9. A method of cosmetic tissue enhancement, said method comprising the steps of administering a cell population according to claim 1 or claim 2 or a pharmaceutical composition according to claim 3 to a patient requiring such enhancement, optionally wherein the cosmetic tissue enhancement is: (a) treating a state associated with cell degeneration or age related tissue change; or (b) limb or organ regrowth or replacement.

10. The cell population of claim 8 or method of claim 9 wherein the cells are: (a) administered intravenously to said patient; or (b) transplanted to the disease site or site of age related degeneration.

11. The cell population or method of any one of claims 8 to 10, wherein the disease is associated with degeneration of pancreatic cells, neuronal cells, cardiovascular cells, epithelial cells, liver cells, muscle cells, retinal cells, hair follicle or kidney cells, for example wherein the disease is diabetes (type I and II), Parkinson's disease, Alzheimer's disease, kidney disease, eye disease, liver disease or cardiovascular disease.

12. The cell population or method of any one of claims 8 to 11 wherein the donor of the cells and the patient are the same species, optionally wherein the patient is human.

13. The cell population of any one of claims 8 to 11 wherein the donor of the cells is rat and the patient is human.

14. A method of producing a specified differentiated cell population, said method comprising the steps of providing a PC population; optionally, selecting a PC cell subpopulation using one or more markers provided in Table 3 ; and culturing said subpopulation of cells in an environment conducive to cell differentiation.

15. A method according to claim 14 further comprising the step of determining suitability for transplantation of given PC cells and derived populations of differentiated and undifferentiated cells for transplantation comprising detecting the presence in the cell population of one or more of the markers provided in Table 4.


SEQUENCE LISTING

<110>  The University Court of the University of Glasgow

<120>  Materials and Methods Relating to Cell Based Therapies

<130>  JEC/FP6755144

<140>  EP 06727122.1
<141>  2006-05-15

<150>  PCT/GB2006/001789
<151>  2006-05-15

<150>  GB 0509748.0
<151>  2005-05-13

<160>  45

<170>  PatentIn version 3.3

<210>  1
<211>  22
<212>  DNA
<213>  Artificial sequence

<220>
<223>  Synthetic sequence: Nestin forward primer

<400>  1
ctagagctgt ttccaggtgc ca                                              22


<210>  2
<211>  20
<212>  DNA
<213>  Artificial sequence

<220>
<223>  Synthetic sequence: NCAM forward primer

<400>  2
cagcgttgga gagtccaaat                                                 20


<210>  3
<211>  20
<212>  DNA
<213>  Artificial sequence

<220>
<223>  Synthetic sequence: cMET forward primer

<400>  3
cattctgctg ctgctgctga                                                 20


<210>  4
<211>  23
<212>  DNA
<213>  Artificial sequence

<220>
<223>  Synthetic sequence: GFAP forward primer

<400>  4
ccctgtctcg aatgactcct cca                                             23

<210>  5
<211>  20
<212>  DNA
<213>  Artificial sequence

<220>
<223>  Synthetic sequence: Ins II forward primer

<400>  5
atggccctgt ggatccgctt                                                    20


<210>  6

<400>  6
000

<210>  7
<211>  22
<212>  DNA
<213>  Artificial sequence

<220>
<223>  Synthetic sequence: Somatostatin forward primer

<400>  7
ctagagctgt ttccaggtgc ca                                                 22


<210>  8
<211>  22
<212>  DNA
<213>  Artificial sequence

<220>
<223>  Synthetic sequence: Pancreatic polypeptide forward

<400>  8
agtccagcag aaggtaggtg tc                                                 22


<210>  9
<211>  18
<212>  DNA
<213>  Artificial sequence

<220>
<223>  Synthetic sequence: Glucagon forward primer

<400>  9
gaccgtttac gtggctgg                                                      18


<210>  10
<211>  20
<212>  DNA
<213>  Artificial sequence

<220>
<223>  Synthetic sequence: PDX-1 forward primer

<400>  10
atcactggag cagggaaggt                                                    20


<210>  11
<211>  20
<212>  DNA
<213>  Artificial sequence

<220>
<223>  Synthetic sequence: CycC Forward

<400>  11
accccaccgt gttcttcgac                                                    20


<210>  12
<211>  18
<212>  DNA
<213>  Artificial sequence

<220>
<223>  Synthetic sequence: Ex2 Forward

<400>  12
gctctcactg gtacagaa                                                      18


<210>  13
<211>  18
<212>  DNA
<213>  Artificial sequence

<220>
<223>  Synthetic sequence: Mbp Forward

<400>  13
ataaccattc cctgcctc                                                      18


<210>  14
<211>  20
<212>  DNA
<213>  Artificial sequence

<220>
<223>  Synthetic sequence: Mobp Forward

<400>  14
acagacagta ttacggtggc                                                    20


<210>  15
<211>  21
<212>  DNA
<213>  Artificial sequence

<220>
<223>  Synthetic sequence: Gtx Forward

<400>  15
gctcctggat aaggatggca a                                                  21


<210>  16
<211>  21
<212>  DNA
<213>  Artificial sequence

<220>
<223>  Synthetic sequence: Myt1 Forward

<400>  16
gcgagaccaa cccacaggac a                                                  21


<210>  17

<211>  20
<212>  DNA
<213>  Artificial sequence

<220>
<223>  Synthetic sequence: Nestin reverse primer

<400>  17
ccagccattt ccttacctgt                                                    20


<210>  18
<211>  20
<212>  DNA
<213>  Artificial sequence

<220>
<223>  Synthetic sequence: NCAM reverse primer

<400>  18
ttaaactcct gtggggttgg                                                    20


<210>  19
<211>  20
<212>  DNA
<213>  Artificial sequence

<220>
<223>  Synthetic sequence: cMET reverse primer

<400>  19
tcactacaca gtcgggacac                                                    20


<210>  20
<211>  23
<212>  DNA
<213>  Artificial sequence

<220>
<223>  Synthetic sequence: GFAP reverse primer

<400>  20
ggagttctcg aacttcctcc tca                                                23


<210>  21
<211>  20
<212>  DNA
<213>  Artificial sequence

<220>
<223>  Synthetic sequence: Ins II reverse primer

<400>  21
tgccaaggtc tgaaggtcac                                                    20


<210>  22

<400>  22
000

<210>  23
<211>  21
<212>  DNA
<213>  Artificial sequence

<220>
<223>  Synthetic sequence: Somatostatin reverse primer

<400>  23
cccagccatt tccttacctg t                                                21


<210>  24
<211>  20
<212>  DNA
<213>  Artificial sequence

<220>
<223>  Synthetic sequence: Pancreatic polypeptide reverse

<400>  24
agttgttgca agagctgggc                                                  20


<210>  25
<211>  24
<212>  DNA
<213>  Artificial sequence

<220>
<223>  Synthetic sequence: Glucagon reverse primer

<400>  25
cggttcctct tggtgttcat caac                                             24


<210>  26
<211>  19
<212>  DNA
<213>  Artificial sequence

<220>
<223>  Synthetic sequence: PDX-1 reverse primer

<400>  26
gctactacgt ttcttatct                                                   19


<210>  27
<211>  20
<212>  DNA
<213>  Artificial sequence

<220>
<223>  Synthetic sequence: CycG Reverse

<400>  27
catttgccat ggacaagatg                                                  20


<210>  28
<211>  28
<212>  DNA
<213>  Artificial sequence

<220>
<223>  Synthetic sequence: Ex2 Reverse

<400>  28
tacattctgg catcagcgca gagactgc                                         28


<210>  29
<211>  19

<212>  DNA
<213>  Artificial sequence

<220>
<223>  Synthetic sequence: Mbp Reverse

<400>  29
ctttttctct tacccccac                                                    19


<210>  30
<211>  20
<212>  DNA
<213>  Artificial sequence

<220>
<223>  Synthetic sequence: Mobp Reverse

<400>  30
gttaccgtag tcgagaagga                                                   20


<210>  31
<211>  20
<212>  DNA
<213>  Artificial sequence

<220>
<223>  Synthetic sequence: Gtx Reverse

<400>  31
ctttttgaga agccgcgtga                                                   20


<210>  32
<211>  22
<212>  DNA
<213>  Artificial sequence

<220>
<223>  Synthetic sequence: Myt1 Reverse

<400>  32
ttacgtggcc ggttccatca ca                                                22


<210>  33
<211>  21
<212>  DNA
<213>  Artificial sequence

<220>
<223>  Synthetic sequence: Ins II nested primer

<400>  33
cctgctcatc ctctgggagc c                                                 21


<210>  34
<211>  22
<212>  DNA
<213>  Artificial sequence

<220>
<223>  Synthetic sequence: Forward primer

<400>  34
acctggttga tcctgccagt ag                                                22

<210>  35
<211>  23
<212>  DNA
<213>  Artificial sequence

<220>
<223>  Synthetic sequence: Reverse primer

<400>  35
agccattcgc agtttcactg tac                                              23


<210>  36
<211>  34
<212>  DNA
<213>  Artificial sequence

<220>
<223>  Synthetic sequence: Probe


<220>
<221>  misc_feature
<222>  (1)..(1)
<223>  Linked to FAM

<220>
<221>  misc_feature
<222>  (34)..(34)
<223>  Linked to TAMRA

<400>  36
tcaaagatta agccatgcat gtctaagtac gcac                                  34


<210>  37
<211>  23
<212>  DNA
<213>  Artificial sequence

<220>
<223>  Synthetic sequence: Forward primer

<400>  37
tttcagcggt tgtaccagtg tct                                              23


<210>  38
<211>  25
<212>  DNA
<213>  Artificial sequence

<220>
<223>  Synthetic sequence: Reverse primer

<400>  38
catattcaaa atgtgctgct gaatt                                            25


<210>  39
<211>  19
<212>  DNA
<213>  Artificial sequence

<220>
<223>  Synthetic sequence: Probe

<220>
<221>  misc_feature
<222>  (1)..(1)
<223>  Linked to FAM

<220>
<221>  misc_feature
<222>  (19)..(19)
<223>  Linked to TAMRA

<400>  39
ctccaggagc ggctgcccc                                                    19


<210>  40
<211>  19
<212>  DNA
<213>  Artificial sequence

<220>
<223>  Synthetic sequence: Forward primer

<400>  40
gagccacagg caccatgtc                                                    19


<210>  41
<211>  21
<212>  DNA
<213>  Artificial sequence

<220>
<223>  Synthetic sequence: Reverse primer

<400>  41
cggcatactt tgctcctgtg t                                                 21


<210>  42
<211>  18
<212>  DNA
<213>  Artificial sequence

<220>
<223>  Synthetic sequence: Probe

<220>
<221>  misc_feature
<222>  (1)..(1)
<223>  Linked to FAM

<220>
<221>  misc_feature
<222>  (18)..(18)
<223>  Linked to TAMRA

<400>  42
cctggtgatg tccgacct                                                     18


<210>  43
<211>  18
<212>  DNA
<213>  Artificial sequence

<220>
<223>  Synthetic sequence: Forward primer

<400>  43

tccctcatca gcaaggca                                                    18


<210>  44
<211>  19
<212>  DNA
<213>  Artificial sequence

<220>
<223>  Synthetic sequence: Reverse primer

<400>  44
gatccgtctg gcctgtctt                                                   19


<210>  45
<211>  18
<212>  DNA
<213>  Artificial sequence

<220>
<223>  Synthetic sequence: Probe

<220>
<221>  misc_feature
<222>  (1)..(1)
<223>  Linked to FAM

<220>
<221>  misc_feature
<222>  (18)..(18)
<223>  Linked to TAMRA

<400>  45
tagccacccc acgactgc                                                    18